# EUROPEAN PATENT SPECIFICATION

(11) **EP 3 881 824 B1**
(45) Date of publication and mention of the grant of the patent: **04.12.2024**
(21) Application number: 20209413.2
(22) Date of filing: 03.10.2014
(51) Int. Cl.: A24B 13/00, A24B 15/00, A61K 31/00, A61K 9/14, A61K 31/375, A61K 31/465, A61K 9/20

(54) **LOZENGE**
LUTSCHTABLETTE
LOSANGE

(30) Priority: 03.10.2013 US 201361886399 P; 03.10.2013 US 201361886391 P; 03.10.2013 US 201361886373 P; 03.10.2013 US 201361886386 P
(43) Date of publication of application: 22.09.2021
(62) Divisional of application: 14790894.1
(73) Proprietor: Altria Client Services LLC, Richmond, Virginia 23230 (US)
(72) Inventor: GAO, Feng, Midlothian, VA Virginia 23114 (US); GEE, Diane L., Chesterfield, VA Virginia 23838 (US); HULAN, Phillip M., Midlothian, VA Virginia 23113 (US); ZHUANG, Shuzhong, Glen Allen, VA Virginia 23060 (US); BURKE, William J., Nashville, TN Tennessee 37206 (US); KOBAL, Gerd, Nashville, TN Tennessee 37203 (US)
(74) Representative: Richardt Patentanwälte PartG mbB

(56) References cited:
- EP-A1- 1 086 691
- EP-A1- 2 177 213
- WO-A1-97/23201

## Description

### CROSS-REFERENCE TO RELATED APPLICATIONS

This application claims priority to U.S. Application Serial No. 61/886,399, filed on October 3, 2013, U.S. Application Serial No. 61/886,391, filed on October 3, 2013, U.S. Application Serial No. 61/886,373, filed on October 3, 2013, U.S. Application Serial No. 61/886,386, filed on October 3, 2013.

### TECHNICAL FIELD

This document relates to lozenges and methods for making lozenges. For example, the lozenge can include nicotine and optionally other ingredients such as exhausted-tobacco fibers, tobacco plant tissue, and/or one or more additives, and one or more additives nicotine within a soluble-fiber matrix (e.g., maltodextrin).

### BACKGROUND

Tobacco can be enjoyed by adult tobacco consumers in a variety of forms. Smoking tobacco is combusted and the aerosol either tasted or inhaled (e.g., in a cigarette, cigar, or pipe). Smokeless tobacco products are not combusted and can include chewing tobacco, moist smokeless tobacco, snus, and dry snuff. Chewing tobacco is coarsely divided tobacco leaf that is typically packaged in a large pouch-like package and used in a plug or twist. Moist smokeless tobacco is a moist, more finely divided tobacco that is provided in loose form or in pouch form and is typically packaged in round cans and used as a pinch or in a pouch placed between an adult tobacco consumer's cheek and gum. Snus is a heat treated smokeless tobacco. Dry snuff is finely ground tobacco that is placed in the mouth or used nasally.

Nicotine is a component of various tobacco products. Over the years, however, various methods and systems have been developed for providing nicotine to adult consumers without the presence of tobacco plant tissue. Some ways tobacco-free nicotine is provided include transdermal patches, lozenges, and nicotine chewing gums.

Nicotine, or 3-(1-methyl-2-pyrrolidinyl) pyridine, is a tertiary amine with the following structure:

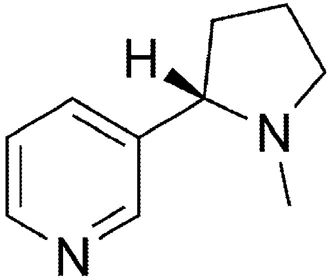

Under ambient conditions, nicotine is an oily, volatile, hygroscopic liquid that is sensitive to light and air. Chemical and physical properties of nicotine present a number of processing and stability issues. For example, because nicotine is volatile, it may evaporate during its incorporation into a gum or lozenge. In an effort to reduce potential processing and stability issues associated with the nicotine compound, a number of nicotine complexes have been developed. For example, one method includes the preparation of a complex of nicotine and an ion exchange resin. A well-known complex that is currently used in the commercially-available nicotine chewing gums is nicotine polacrilex, which is a complex of nicotine and the cation exchange resin AMBERLITE 164.

Oral products providing flavor and/or one or more active ingredients are well known. One such oral product is chewing gum. Other oral products include hard pieces (e.g., mints). Softer gelatin-based oral products are also known. Pharmaceutical and therapeutic products (e.g., cough-suppressant lozenges) can also be provided in a solid form for oral consumption. The flavor release and/or active agent release characteristics for an oral product are important for providing an improved consumer product.

Patent document EP2177213 generally describes a dust-reduced, nicotine-containing granulate including 1-50 weight percent of nicotine or a pharmaceutically acceptable nicotine derivative and 50-99 weight percent of an excipient.

WO97/23201 generally describes a chewable tablet or lozenge for delivering soluble dietary fibers. According to the WO97/23202 the chewable tablet may also include an excipient and/or additives such as vitamins, minerals, coloring agents, or lubricants/release agents.

EP1086691 generally describes a tablet or lozenge for supplying carbohydrate over a long period of time.

EP2177213, WO97/23201, and EP1086691 when considered individually or in combination do not disclose, teach, or suggest the claimed features as recited in the independent claims.

### SUMMARY

A lozenge provided herein provides a satisfying tactile and/or flavor experience. The lozenge according to the invention is set out in the appended set of claims. A lozenge provided herein includes a body that is wholly receivable in an oral cavity. According to an aspect of the invention, the body has a glass transition temperature ranging from 50°C to 120°C and includes, a matrix including, amorphous, non-cross-linked soluble fibers present in the body in an amount greater than or equal to 40 weight percent of the body, a plasticizer in an amount ranging from 0.05 weight percent to 10 weight percent, water in an amount ranging from 2 weight percent to 15 weight percent, and an additive dispersed in the matrix. The plasticizer is dispersed in the matrix. The plasticizer includes propylene glycol, triacetin, glycerin, vegetable oil, partially hydrogenated oil, triglycerides, or any combination thereof. The body is free of cellulosic fibers.

The body can include a soluble-fiber matrix with exhausted-tobacco, plant tissue fiber, and one or more additives (e.g., nicotine or a derivative thereof) dispersed in the soluble-fiber matrix). In some cases, the body can include unbound nicotine in solution with a soluble fiber matrix and/or optionally absorbed into other ingredients, such as exhausted-tobacco fiber. In some cases, a lozenge provided herein includes at least 40 weight percent of amorphous, non-cross-linked soluble fiber. In some cases, a lozenge provided herein can include maltodextrin. A lozenge provided herein can be adapted to release the nicotine or a derivative thereof or other optional ingredients such as tobacco plant tissue from the body when the body is received within the oral cavity of an adult tobacco consumer and exposed to saliva. A body of a lozenge provided herein can be a single phase structure. In some cases, the body of a lozenge provided herein can be amorphous.

Soluble fiber lozenges provided herein can, in some cases, include at least 1 weight percent tobacco plant tissue. A lozenge provided herein can, in some cases, include 5 weight percent tobacco plant tissue. A lozenge provided herein can, in some cases, include at least 10 weight percent tobacco plant tissue. A lozenge provided herein can, in some cases, include 20 weight percent tobacco plant tissue. A lozenge provided herein can, in some cases, include 30 weight percent tobacco plant tissue. A lozenge provided herein can, in some cases, include at least 40 weight percent tobacco plant tissue. In some cases, tobacco plant tissue used in a lozenge provided herein can be processed to have an average particle size. In some cases, one or more additives can be selected from the categories of flavorants, sweeteners, vitamins, minerals, therapeutic agents, nutraceuticals, energizing agents, soothing agents, coloring agents, amino acids, chemesthic agents, antioxidants, food grade emulsifiers, pH modifiers, botanicals, teeth whitening agents, and/or alkaloids (e.g., caffeine). In some cases, a lozenge provided herein can have various fiber lengths such as combinations of less than 200 micrometers, less than 150 micrometers, less than 125 micrometers, less than 100 micrometers, less than 75 micrometers, less than 50 micrometers, less than 25 micrometers, less than 20 micrometers, or less than 10 micrometers. In some cases, tobacco plant tissue used in a lozenge provided herein can be processed to have an average particle size of at least 1 micrometer, at least 5 micrometers, at least 10 micrometers, at least 25 micrometers, at least 50 micrometers, at least 75 micrometers, at least 100 micrometers, at least 125 micrometers, or at least 150 micrometers. In some cases, tobacco plant tissue used in a lozenge provided herein can be processed to have an average particle size of between 25 and 125 micrometers. According to another aspect of the invention, a lozenge is provided which comprises:
a non-porous body that is wholly receivable in an oral cavity, the body including,
a matrix including,
   an amount greater than or equal to 40 weight percent of amorphous, non-cross-linked, amorphous, non-cross-linked, digestion resistant soluble fibers,
   water in an amount ranging from 2 weight percent to 15 weight percent of the body, and
   a plasticizer in an amount ranging from 0.5 weight percent to 10 weight percent, the plasticizer being dispersed in the matrix, and the plasticizer including propylene glycol, triacetin, glycerin, vegetable oil, partially hydrogenated oil, triglycerides, triacetin, or any combination thereof; and
an additive dispersed in the matrix.

A method of making lozenges provided herein can include forming a molten mixture of at least 40 weight percent soluble fiber, 1 weight percent of tobacco, exhausted-tobacco fiber, one or more additives (e.g., nicotine or a derivative thereof), and less than 15 weight percent water, while maintaining a mixture temperature of less than 150 °C. The molten mixture is then portioned into a plurality of lozenges. In some cases, the mixture temperature is less than 180 °C. In other cases, the mixture is maintained at a temperature of less than 200 °C. In some cases, the ingredients used to form the molten mixture can be mixed in an extruder, flattened into a sheet of a predetermined thickness as it leaves the extruder, and cut into individual lozenges from the sheet before the sheet cools below the glass transition temperature range of the molten mixture. Unlike a traditional lozenge, which incorporates sugars or sugar alcohols that are heated to a temperature such that caramelization occurs, methods provided herein include heating the molten mixture of nicotine or a derivative thereof, water, the soluble fiber, and optionally other ingredients such as exhausted-tobacco fibers, tobacco plant tissue, and/or one or more additives to form a solution or a dispersion without significant crosslinking occurring. In some cases, additional additives can be added that are dispersed within the soluble fiber matrix, but not in solution with the soluble fiber. Degradation of nicotine and certain additives (e.g., therapeutic agents) can be accelerated when exposed to elevated temperatures over an extended period of time, therefore, the temperatures of the molten mixture provided herein can be maintained at a temperature of 150 °C or below over a residence time of 5 to 10 minutes or less (for example if an extrusion process is utilized). In some cases, the temperatures can be maintained at a temperature of 200 °C or below over a residence time of 5 to 10 minutes because tobacco plant tissue can have negative sensorial characteristics and the degradation of certain additives (e.g., therapeutic agents) can be accelerated when exposed to temperatures in excess of 200 °C over an extended period of time.

In some cases, a molten mixture provided herein is heated to a temperature of between 80 °C and 150°C. In some cases, a molten mixture provided herein is heated to a temperature of between 80 °C and 200 °C. In some cases, a molten mixture provided herein is heated to a temperature of between 100 °C and 110 °C. In some cases, a molten mixture provided herein is heated to a temperature of between 80 °C and 110 °C. When cooled below its glass transition temperature, a molten mixture provided herein solidifies into an amorphous, non-porous, soluble fiber matrix containing nicotine and optionally one or more ingredients, such as tobacco particles, exhausted tobacco, one or more additives, or other ingredients. The soluble fibers do not become crosslinked, therefore, the soluble fibers remain soluble and thus dissolve when placed in an adult tobacco consumer consumer's mouth.

A lozenge body can be rigid and brittle. In some cases, a body provided herein can have a glass transition temperature greater than 37 °C. In some cases, a body provided herein can have a glass transition temperature of between 50 °C and 120 °C. In some cases, a body provided herein can have a glass transition temperature of between 80 °C and 100 °C. In some cases, a body provided herein having 90 weight percent maltodextrin can have a glass transition temperature of approximately 98°C. A lozenge provided herein can have a coating over the body. In some cases, the body of a lozenge provided herein can be non-porous.

A lozenge body can include at least 40 weight percent of amorphous, non-cross-linked soluble fiber. In some cases, the lozenge body includes at least 50 weight percent of amorphous, non-cross-linked soluble fiber. In some cases, the lozenge body includes at least 60 weight percent of amorphous, non-cross-linked soluble fiber. In some cases, the lozenge body includes at least 70 weight percent of amorphous, non-cross-linked soluble fiber. In some cases, the lozenge body includes at least 75 weight percent of amorphous, non-cross-linked soluble fiber. In some cases, the lozenge body includes at least 80 weight percent of amorphous, non-cross-linked soluble fiber. In some cases, the lozenge body includes at least 85 weight percent of amorphous, non-cross-linked soluble fiber. In some cases, the lozenge body includes at least 90 weight percent of amorphous, non-cross-linked soluble fiber. In some cases, the lozenge body includes at least 95 weight percent of amorphous, non-cross-linked soluble fiber. In some cases, the soluble fiber can include maltodextrin, psyllium, inulin, arabinoxylans, cellulose, and many other plant components, such as resistant starch, resistant dextrins, lignin, pectins, beta-glucans, and oligosaccharides, or a combination thereof. In some cases, a lozenge body can include at least 40 weight percent maltodextrin. In some cases, the lozenge body includes at least 50 weight percent maltodextrin. In some cases, the lozenge body includes at least 60 weight percent maltodextrin. In some cases, the lozenge body includes at least 70 weight percent maltodextrin. In some cases, the lozenge body includes at least 75 weight percent maltodextrin. In some cases, the lozenge body includes at least 80 weight percent maltodextrin. In some cases, the lozenge body includes at least 85 weight percent maltodextrin. In some cases, the lozenge body includes at least 90 weight percent maltodextrin. In some cases, the lozenge body includes at least 95 weight percent maltodextrin.

In some cases, a lozenge can include maltodextrin, psyllium, inulin, arabinoxylans, cellulose, and many other plant components, such as resistant starch, resistant dextrins, lignin, pectins, beta-glucans, and oligosaccharides, or a combination thereof.

In some cases, a lozenge provided herein can include amorphous, non-cross-linked, digestion resistant soluble fiber (e.g., maltodextrins). Suitable maltodextrins include those that are soluble in water up to 70% at 20° C, have a viscosity of about 15 cps for a 30% solution at 30° C, a DE in the range of about 6-16, and contain random α-1,2, α-1,3, α-1,4, β -1,2, β -1,3 and β -1,4 glucose linkages in addition to the normal α-1,4 glucose linkages found in partially hydrolyzed starch. See, for example., US Patent Nos. 5,410,035; 5,380,717. For example, Fibersol^{®}-2 is a maltodextrin of DE 6-10 processed from corn starch using hydrochloric acid and enzymes, which can be used as the soluble fiber in a lozenge provided herein. Fibersol^{®}-2 is partially indigestible because human digestive enzymes are incapable of digesting β 1,2, β 1,3 and β 1,6 glucose bonds. See, for example, US Patent No. 6,203,842. Other starch sources, such as potato, rice, wheat, barley, peas, beans, lentils, oats, or tapioca, can be processed to form amorphous, non-cross-linked, digestion resistant soluble fiber. A digestion resistant soluble fiber includes starch linkages that cannot be hydrolyzed by enzymes of the human digestive tract. Soluble fiber used in a lozenge provided herein can be a soluble fiber generally recognized as safe ("GRAS") by the Food and Drug Administration or another appropriate private, state, or national regulatory agency.

A lozenge provided herein can, in some cases, include up to 15 weight percent water. In some cases, a lozenge provided herein can include between 2 weight percent and 15 weight percent water. In some cases, a lozenge provided herein can include between 3 weight percent and 10 weight percent water. In some cases, a lozenge provided herein can include between 4 weight percent and 7 weight percent water.

Nicotine or derivatives thereof added to a lozenge provided herein can be in any suitable form. In some cases, a lozenge provided herein includes between 0.1 mg and 20 mg nicotine. In some cases, a lozenge provided herein includes between 0.5 mg and 10 mg nicotine. In some cases, a lozenge provided herein includes between 1.0 mg and 3.0 mg nicotine. In some cases, a lozenge provided herein includes tobacco-derived nicotine. In some cases, a lozenge provided herein includes synthetic nicotine. A lozenge provided herein, in some cases, can be free of tobacco plant tissue. A lozenge provided herein can be free of cellulose fibers. In some cases, a lozenge provided herein includes less than 40 weight percent of exhausted-tobacco fiber. For example, in some cases, a lozenge provided herein includes between 0.5 weight percent and 40 weight percent of exhausted-tobacco fiber. In some cases, a lozenge provided herein includes between 1.0 weight percent and 10 weight percent of exhausted-tobacco fiber and/or cellulose fiber.

A lozenge provided herein can include a sweetener dispersed therein. Suitable sweeteners include saccharine, sucralose, aspartame, acesulfame potassium, and combinations thereof. In some cases, a lozenge provided herein can be free of sugars and sugar alcohols. For example, a lozenge can be free of sugars and sugar alcohols, but include non-nutritive sweeteners. In some cases, a lozenge provided herein can include non-caramelized sugars and/or sugar alcohols in a percentage of no more than 25 weight percent. For example, mannitol and/or sorbitol can be added to reduce the glass transition temperature of a molten mixture provided herein. When included, sugars and sugar alcohols in a molten mixture form a solution with the soluble fiber. Sugars and sugar alcohols can alter the glass transition temperature of a molten mixture provided herein. When cooled below the glass transition temperature, a solution of amorphous, non-cross-linked soluble fiber and sugar alcohols remains a single-phase, non-crosslinked structure. In other cases, when cooled below the glass transition temperature, a solution of amorphous, non-cross-linked soluble fiber and sugar alcohols remains an amorphous, non-crosslinked structure

A lozenge provided herein can include one or more flavorants as an additive. The flavorants can be natural or artificial. Flavorants can be selected from the following: licorice, wintergreen, cherry and berry type flavorants, Drambuie, bourbon, scotch, whiskey, spearmint, peppermint, lavender, cinnamon, cardamom, apium graveolents, clove, cascarilla, nutmeg, sandalwood, bergamot, geranium, honey essence, rose oil, vanilla, lemon oil, orange oil, Japanese mint, cassia, caraway, cognac, jasmin, chamomile, menthol, ylang ylang, sage, fennel, pimenta, ginger, chai, anise, chai, coriander, coffee, mint oils from a species of the genus Mentha, cocoa, and combinations thereof. Synthetic flavorants can also be used. The particular combination of flavorants can be selected from flavorants that are GRAS in a particular country, such as the United States. Flavorants can also be included in the lozenge as encapsulated flavorants.

A lozenge provided herein can include a plasticizer dispersed in the soluble-fiber matrix. For example, the plasticizer can be propylene glycol, triacetin, glycerin, vegetable oil, partially hydrogenated oil, triglycerides, or a combination thereof.

A body of a lozenge provided herein can have a variety of different shapes, some of which include disk, shield, heart, rectangle, and square. In some cases, a body of a lozenge provided herein can have rounded corners. In some cases, the body of the lozenge can be spherical. According to certain cases, the body can have a length or width of between 1 mm and 25 mm and a thickness of between 1 mm and 25 mm. In some cases, the body can have a length or width of between 5 mm and 15 mm and a thickness of between 2 mm and 5 mm.

In some cases, a lozenge provided herein can include a colorant. For example, a body of a lozenge provided herein can include titanium dioxide, which can provide the body with a white color. In some cases, a coating on the body can include a colorant.

A method of forming lozenges can include forming a molten mixture of at least 40 weight percent soluble fiber, nicotine, and less than 15 weight percent water, while maintaining a mixture temperature of less than 200 °C, less than 150 °C, or less than 130°C. In some cases, other ingredients, such as exhausted tobacco fiber, tobacco particles, and/or one or more additives, can be dispersed within that molten mixture. The one or more additives can include one or more additives selected from colorants, sweeteners, flavorants, plasticizers, antioxidants, and combinations thereof. In some cases, the molten mixture is free of cellulose fiber, tobacco plant tissue, sugar, and/or sugar alcohols. In some cases, the molten mixture includes at less than 13 weight percent, less than 10 weight percent, less than 8 weight percent, less than 7 weight percent, less than 6 weight percent, or less than 5 weight percent water. In some cases, the molten mixture includes at least 3 weight percent, at least 4 weight percent, at least 6 weight percent, or at least 7 weight percent water.

In some cases, the molten mixture provided herein is formed in an extruder. The extruder can be a multi-staged extruder having different sections that are heated to different temperatures and/or have different ingredients introduced. In some cases, an extruder provided herein can include multiple stages and can be used in a method provided herein in a process where the maximum temperature in any stage is no more than 200 °C, (e.g., no more than 150 °C, no more than 130 °C, no more than 120 °C, no more than 110 °C, no more than 105 °C). In some cases, the molten mixture can be heated to a maximum temperature of greater than the molten mixture's Tg and less than 150°C. In some cases, the molten mixture can be heated to a maximum temperature of greater than the molten mixture's Tg and less than 200 °C.

Portioning the molten mixture provided herein can be accomplished using any suitable method. In some cases, the molten mixture can be formed into a sheet of a predetermined thickness as it comes out of the extruder and individual lozenges cut from the sheet with a stamping die. A method provided herein can further include cooling lozenges and packaging lozenges.

The details of one or more embodiments of the subject matter described in this specification are set forth in the accompanying drawings and the description below. Other features, aspects, and advantages of the subject matter will become apparent from the description, the drawings, and the claims.

### BRIEF DESCRIPTION OF THE DRAWINGS

Figure 1 is a perspective view of an exemplary lozenge provided herein.
Figures 1A-1O illustrates various exemplary shapes of lozenges provided herein.
Figure 2 is a differential scanning calorimetry data for an exemplary molten mixture provided herein.
Figure 2A is a modulated differential scanning calorimetry data for an exemplary molten mixture provided herein.
Figures 3A-3D depicts X-ray microtomography images from an exemplary extruded lozenge provided herein.
Figure 4 is a dynamic mechanical analysis (DMA) of an exemplary extruded lozenge provided herein.
Figure 5 depicts an exemplary process flow diagram for making lozenges provided herein.

### DETAILED DESCRIPTION

The lozenges described herein can include nicotine or a derivative thereof and optionally additional ingredients, such as exhausted-tobacco fibers, tobacco plant tissue, and/or one or more additives in a soluble-fiber matrix. A body of a lozenge provided herein can include a soluble fiber phase forming a matrix around one or more ingredients. Nicotine or a derivative thereof or one of the additional ingredients can be dispersed in the soluble-fiber matrix such that the nicotine or derivative thereof or one of the additional ingredients is released from the lozenge as it dissolves when the lozenge is received within the oral cavity of an adult tobacco consumer and exposed to saliva. The lozenges described herein can provide a favorable additive release profile and tactile experience. In some cases, a lozenge provided herein includes unbound nicotine in solution with soluble fiber of the matrix.

A lozenge provided herein, in some cases, include one or more additives selected from the categories of flavorants, sweeteners, vitamins, minerals, therapeutic agents, nutraceuticals, energizing agents, soothing agents, coloring agents, amino acids, chemesthic agents, antioxidants, food grade emulsifiers, pH modifiers, botanicals, teeth whitening agents, and/or alkaloids (e.g., caffeine). Combinations of additives (e.g., sweeteners, flavorants, and caffeine) can be combined to provide a favorable tactile and flavor experience.

A lozenge provided herein can take up to 1 hour to dissolve when placed in an adult consumer's mouth. In some cases, a lozenge provided herein can take between 1 minute and 30 minutes to dissolve when placed in an adult consumer's mouth if the adult tobacco consumer does not masticate the lozenge. In some cases, a lozenge provided herein can take between 2 minutes and 15 minutes to dissolve when placed in an adult consumer's mouth if the adult consumer does not masticate the lozenge.

In some cases, a lozenge can be free of tobacco plant tissue. As used herein, the term "tobacco plant tissue" refers to processed or non-processed cellulosic parts (e.g., leaves, stems) of a member of the genus *Nicotiana,* but does not include extracts of tobacco (e.g., tobacco-derived nicotine). As used herein, "free of tobacco plant tissue" means that the product includes less than 0.5 weight percent of tobacco plant tissue. For example, a lozenge provided herein can include one or more organoleptic components extracted from raw or processed tobacco, yet be free of tobacco plant tissue. In some cases, a lozenge provided herein can include one or more organoleptic components extracted from raw or processed tobacco, yet include no tobacco plant tissue.

Lozenges provided herein can be free of cellulose fibers. In some cases, a lozenge provided herein includes up to 10 weight percent cellulosic fibers. Cellulosic fibers used in a lozenge provided herein can have an average fiber length of less than 200 micrometers. In particular cases, cellulosic fibers in a lozenge provided herein have lengths between 25 and 125 micrometers.

In addition to nicotine, sweeteners, flavorants, and other optional ingredients, such as exhausted-tobacco fibers, tobacco plant tissue, and/or one or more additives, the lozenge can also include fillers, plasticizers, antioxidants, colorants, and/or processing aids. Fillers can also be included in the soluble-fiber matrix to alter the texture or pliability of the lozenge. The soluble-fiber matrix can also include plasticizers (e.g., propylene glycol), which can increase the softness of a lozenge provided herein. Antioxidants can be used to preserve nicotine in the lozenge. Processing aids can also be present in the lozenge and be used to facilitate shaping processes.

### Lozenge Shapes and Packaging

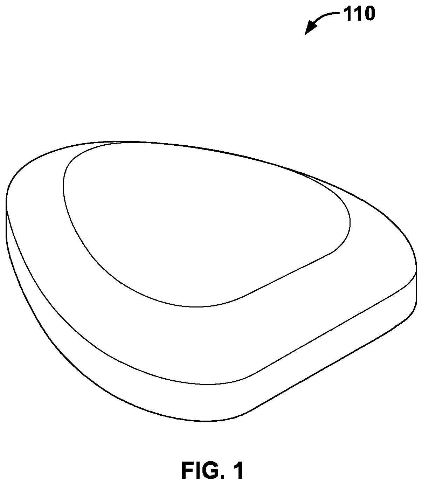

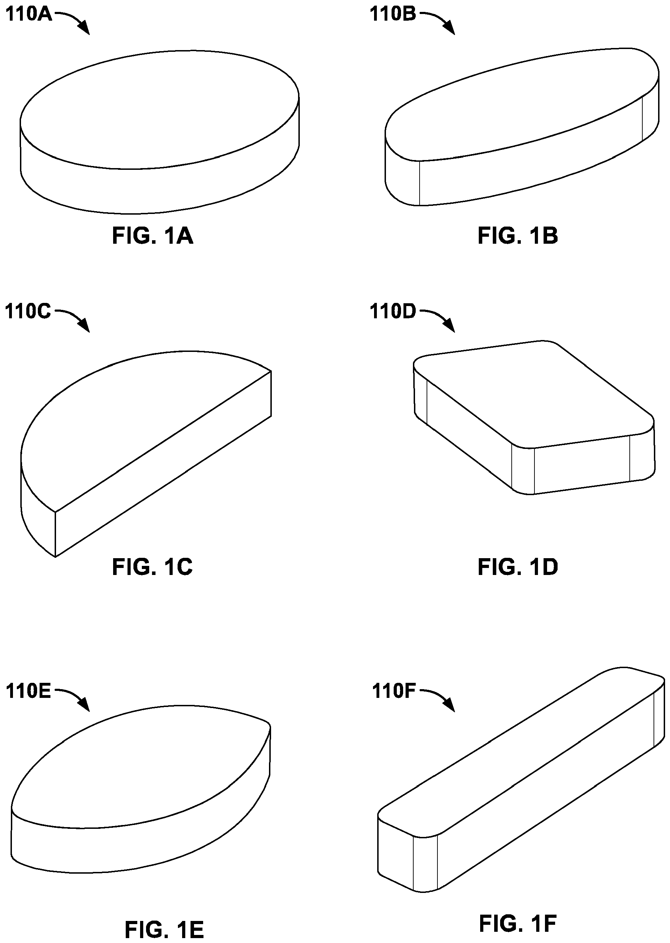

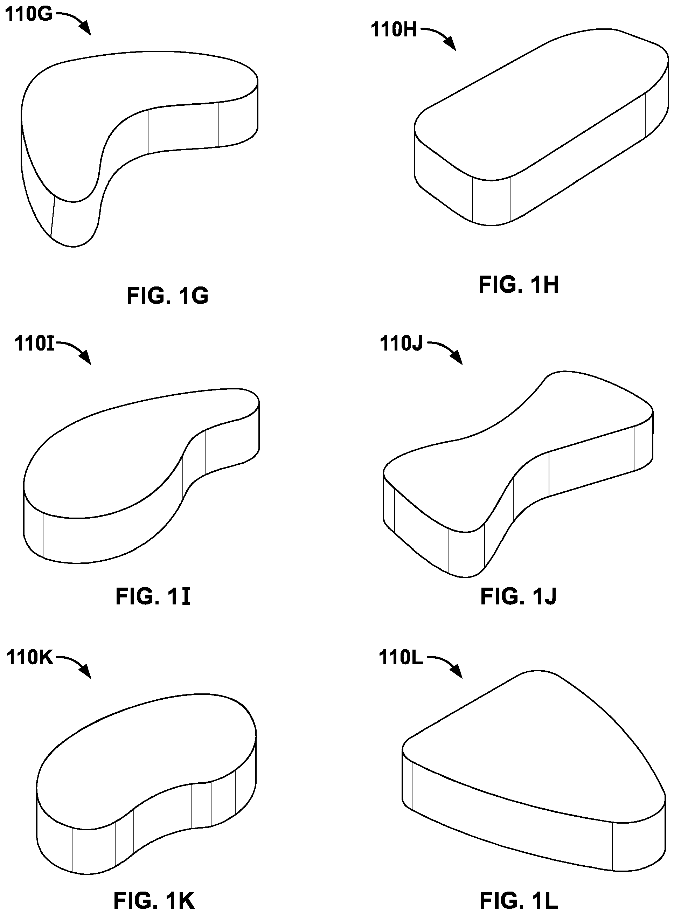

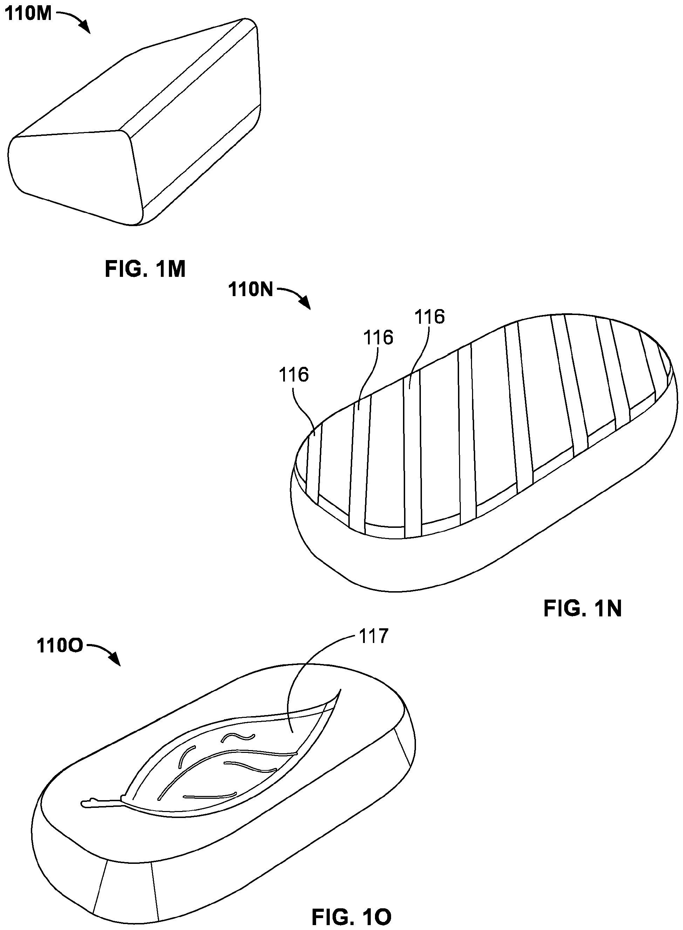

Figure 1 depicts an example of a lozenge 110. The lozenge 110 has a rounded shield shape. For example, lozenge 110 can have a diameter of about 16 mm, a width of 14 mm, and a thickness of about 11 mm. In some cases the lozenge can have a rounded shape. For example, the lozenge can have a diameter of about 12 mm, a width of 14 mm, and a thickness of about 2.5 mm.

Referring now to Figures 1A-1N, lozenges provided herein can be molded into any desired shape. For example, referring to Figures 1A-1L, lozenges 110A-L can be formed in shapes that promotes improved positioning in the oral cavity, improved packaging characteristics, or both. In some circumstances, lozenges 110A-L can be configured to be (A) an elliptical-shaped lozenge 110A; (B) an elongated elliptical-shaped lozenge 110B; (C) semi-circular lozenge 110C; (D) square or rectangular-shaped lozenge 110D; (E) football-shaped lozenge 110E; (F) elongated rectangular-shaped lozenge 110F; (G) boomerang-shaped lozenge 110G; (H) rounded-edge rectangular-shaped lozenge 110H; (I) teardrop- or comma-shaped lozenge 110I; (J) bowtie-shaped lozenge 110J; (K) peanut-shaped lozenge 110K; and (L) flat shield-shaped lozenge. Alternatively, the lozenge can have different thicknesses or dimensionality, such that a beveled article (e.g., a wedge) is produced (see, for example, product 110M depicted in Figure 1M) or a hemi-spherical shape is produced.

In addition, or in the alternative to flavorants being included within the soluble-fiber matrix, flavorants can be included on an exterior of the lozenge 110. For example, referring to Figure 1N, some embodiments of a lozenge 110N can be equipped with flavor strips 116.

Referring to Figure 1O, particular embodiments of the lozenge 110 can be embossed or stamped with a design (e.g., a logo, an image, or the like). For example, the lozenge 110O can be embossed or stamped with any type of design 117 including, but not limited to, a trademark, a product name, or any type of image. The design 117 can be formed directly into the lozenge, arranged along the exterior of the product 110O. The design 117 can also be embossed or stamped into those embodiments with a dissolvable film 116 applied thereto.

In some cases, the lozenge 110 or lozenges 110A-O can be wrapped or coated in an edible or dissolvable film, which may be opaque, substantially transparent, or translucent. The dissolvable film can readily dissipate when the lozenge 110 is placed in an oral cavity. In some cases, the lozenge 110 can be coated with a mouth-stable material. Exemplary coating materials include, Carnauba wax, Beeswax, gelatin, acetylated monoglyceride, starch (e.g., native potato starch, high amylose starch, hydroxypropylated potato starch), Zein, Shellac, ethyl cellulose, methylcellulose, hydroxypropyl methylcellulose, carboxymethyl cellulose, and combinations thereof. Additives, such as miglycol, titanium dioxide, kaoline, bentonite, can be incorporated into the coating material to improve oxygen or moisture barrier and mechanical properties for the coating or film. For example, a coating can include a combination of gelatin and methylcellulose or gelatin and hydroxymethylcellulose. In some cases, the coating can contain sugar alcohols (such as sorbitol, mannitol, xylitol, erythritol), disaccharide-derived (e.g., isomalt, lactitol, maltitol), or polysaccharide-derived mixtures (e.g., maltitol syrup, hydrogenated starch hydrolysates [HSH]) or combinations thereof. In some cases, a coating material can contain sugar alcohols and hydroxyethyl cellulose, gelatin, wax, with additives. For example, a coating can include a combination of gelatin and methylcellulose. In some cases, a coating material can include a plasticizer. In some cases, a coating can include a colorant, a flavorant, and/or one or more of the additives discussed above. For example, a coating can include nicotine to provide a user with readily available nicotine. In some cases, the body of a lozenge provided herein can have surfaces roughened to improve the adherence of a coating. In some cases, a coating can provide a glossy or semi-glossy appearance, a smooth surface, and/or an appealing visual aesthetic (e.g., a nice color). In some cases, the coating (e.g., a Beeswax, Zein, acetylated monoglyceride, and/or hydroxypropylated potato starch coating) can provide a soft mouth feel. In some cases, the coating (e.g., a methylcellulose, hydroxypropyl methylcellulose, carboxymethyl cellulose, ethyl cellulose, and/or gelatin coating) can provide a hard outer coating.

One or more lozenges 110 can be packaged in a variety of conventional and non-conventional manners. For example, a plurality of lozenges 110 can be packaged in a container having a lid. In some cases, a plurality of lozenges 110 can be stacked and packaged in a paper, plastic, and/or aluminum foil tube. The packaging can have a child-resistant lid.

### Lozenge Properties

The lozenge 110 can provide a favorable tactile experience (e.g., feeling in the mouth). The lozenge 110 can also retain its shape during processing, shipping, handling, and optionally while placed in the adult consumer's mouth. In some cases, the lozenge 110 can be rigid. In some cases, a lozenge 110 can be brittle such that an adult consumer can crunch or masticate the lozenge 110 in the adult consumer's mouth. A lozenge 110 provided herein can be non-porous. Manipulation of a lozenge 110 provided herein to increase the exposure of surfaces to saliva can accelerate a dissolution rate.

A lozenge 110 provided herein can have a glass transition temperature (Tg) that is in the range of 50 °C to 120 °C (i.e., about 122 °F to about 248 °F), depending on the formulations (e.g., soluble fiber type and weight percentage, water content, total flavor weight percentage, etc.) and processing conditions used to form the lozenge 110. The Tg can impact the preferred operating temperature used to form a solution of the soluble fiber, nicotine, and other ingredients. By changing the soluble fiber weight percentage and type, the Tg range can be altered. In some cases, when a lozenge provided herein is placed in an adult consumer's mouth, the lozenge is not soft but remains as an amorphous glassy state, as the adult consumer's body temperature is below the glass transition temperature range of the product.

The lozenges provided herein can remain in a glassy state throughout the duration of their shelf life (e.g., at least 2 months, at least 6 months, at least 1 year, or at least 2 years). The Tg temperature can also impact a sensorial experience provided by a lozenge provided herein. For example, a glass transition temperature above body temperature can impede a lozenge from becoming sticky when placed in the adult consumers' mouth.

A lozenge 110 provided herein can have any desirable color. In some cases, a lozenge 110 provided herein can be translucent and have an off-white color. In some cases, a colorant can be included to provide a desired visual appearance. In some cases, natural and artificial colorants can be added to a soluble-fiber matrix of a lozenge 110. In some cases, colorants can make a body of a lozenge opaque. For example, titanium dioxide can be added to a soluble-fiber matrix to produce an opaque white lozenge. Encapsulated flavors can be added during the extrusion process to create speckles, patterns or dots within the lozenge or on a surface of a lozenge 110. In some cases, a coating applied to a body of a lozenge can provide a desirable color.

The lozenge as described herein was made using about 80-85 weight percent Fibersol^{®}-2 digestion resistant maltodextrin, between 6-7 weight percent water, and other ingredients that included nicotine, flavors, sweeteners, antioxidants, colorant, plasticizer, and processing aides. A test of the lozenge demonstrated that nicotine degradants and impurities after sixteen weeks were non-detectable. For example, as shown in Table 1, all nicotine degradants at week 16 were non-detectable under controlled conditions of 25 °C, 65% relative humidity, and atmospheric pressure.

**Table 1**

| % Relative to Nicotine Concentration of Lozenge at week 16 (Average, n=3) | | | | | | |
|---|---|---|---|---|---|---|
| Myosmi ne | Nornicoti ne | Anabasi ne | Cotini ne | Anatabi ne | Nicotin e-Oxide | b-Nicotyri ne |
| ND | ND | ND | ND | ND | ND | ND |

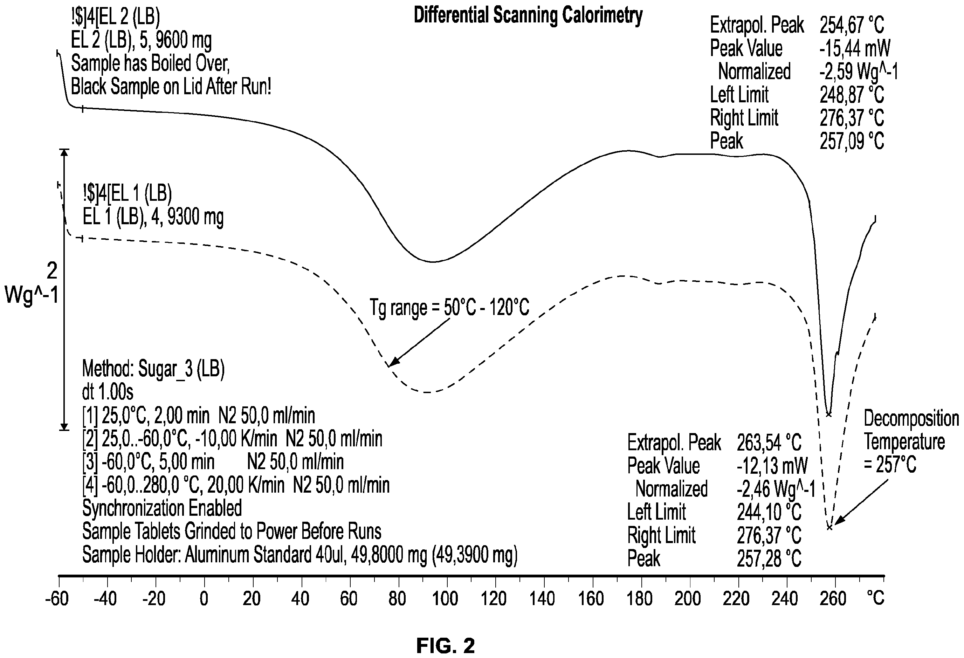

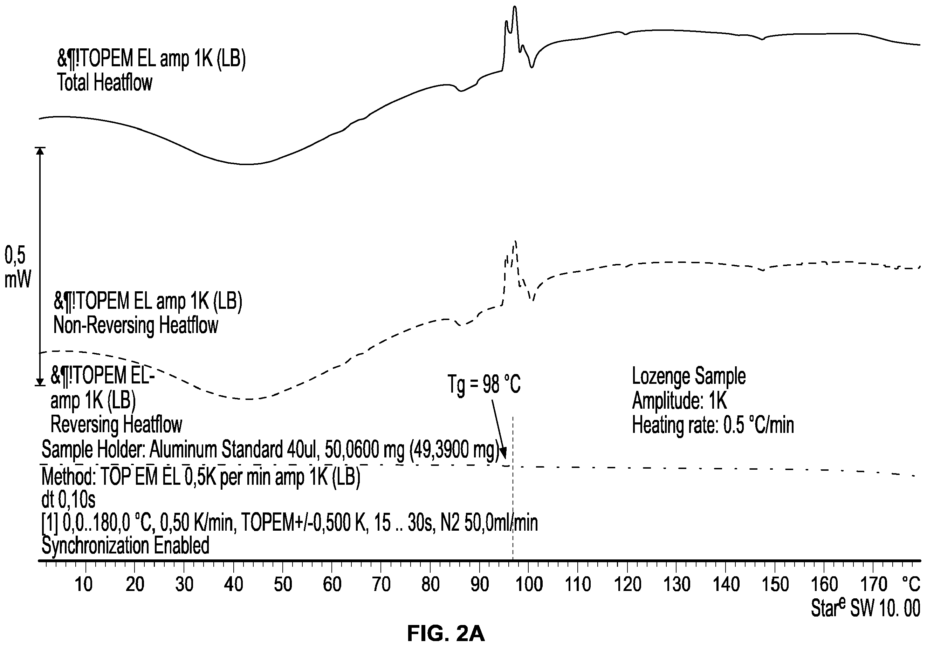

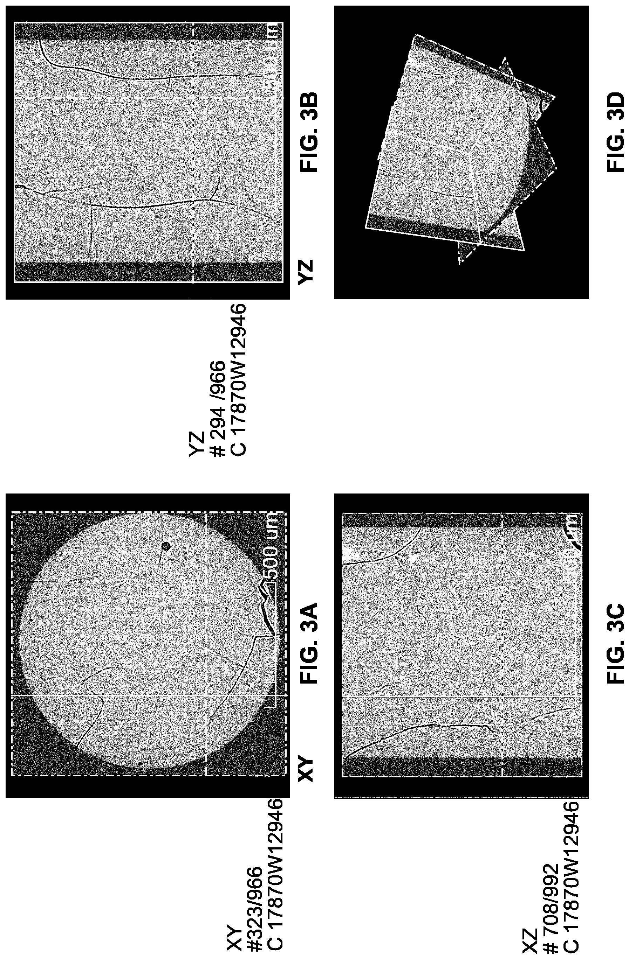

Figure 2 shows a differential scanning calorimetry (DSC) data for an exemplary molten mixture provided herein, a lozenge that has a Tg range of between 50 °C and 120 °C. By having the lower limit of the Tg range higher than body temperature (i.e., about 37 °C), lozenges provided here can remain rigid and non-sticky when placed in an adult consumer's mouth. A Tg temperature for a particular molten mixture can guide a preferred operating temperature range for the extrusion process. The process is designed to stay well below the decomposition temperature of ~257°C as measured by DSC (See Figure 2). One component of the mixtures that affects Tg range is the soluble fiber weight percentage and type. For example, Figure 2A depicts the modulated DSC for an exemplary mixture, provided herein, which shows a Tg for a lozenge body that has a Tg of 98 °C. By changing the soluble fiber weight percentage and type, the Tg range can be altered. When a lozenge provided herein is placed in an adult consumer's mouth, the lozenge can remain as an amorphous glassy state, as the adult consumer's body temperature is below the glass transition temperature range of the lozenge provided herein. A lozenge provided herein can be designed to remain in a glassy state throughout the duration of its shelf life for the product. In some cases, a lozenge provided herein can have a Tg that impacts the sensorial experience. For example, a lozenge provided herein having a Tg range greater than body temperature and can remain non-sticky when placed in an adult consumer's mouth. The structure can be non-porous. Figures 3A-3D depict X-ray microtomography images from an exemplary extruded lozenge provided herein, which shows that the structure is non-porous.

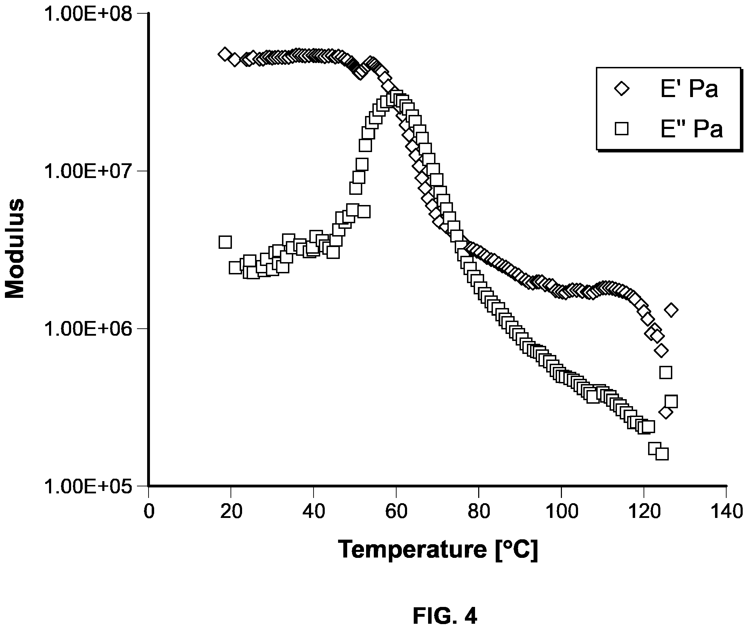

Figure 4 is a dynamic mechanical analysis (DMA) of an exemplary extruded lozenge provided herein. The storage modulus (blue line, diamond legend) is correlated to the stiffness (E') of the material and this informs where the mixture viscosity is sufficiently pliable for processing (i.e., rubbery state). The extrusion temperature operating range is between 100 °C and 150 °C.

### Nicotine

Nicotine within a lozenge provided herein can be tobacco-derived nicotine, synthetic nicotine, or a combination thereof. In some cases, the nicotine can be liquid nicotine. Liquid nicotine can be purchased from commercial sources, whether tobacco-derived or synthetic. In some cases, a lozenge provided herein includes between 0.1 mg and 20.0 mg of nicotine. In some cases, a lozenge provided herein includes between 0.5 mg and 10.0 mg of nicotine. In some cases, a lozenge provided herein includes between 1.0 mg and 3.0 mg of nicotine.

Tobacco-derived nicotine can include one or more other tobacco organoleptic components other than nicotine. The tobacco-derived nicotine can be extracted from raw (e.g., green leaf) tobacco and/or processed tobacco. Processed tobaccos can include fermented and unfermented tobaccos, dark air-cured, dark fire cured, burley, flue cured, and cigar filler or wrapper, as well as the products from the whole leaf stemming operation. The tobacco can also be conditioned by heating, sweating and/or pasteurizing steps, as described in U.S. Publication Nos. 2004/0118422 or 2005/0178398. Fermenting typically is characterized by high initial moisture content, heat generation, and a 10 to 200 loss of dry weight. *See, e.g.,* U.S. Patent Nos. 4,528,993; 4,660,577; 4,848,373; and 5,372,149. By processing the tobacco prior to extracting nicotine and other organoleptic components, the tobacco-derived nicotine may include ingredients that provide a favorable experience. The tobacco-derived nicotine can be obtained by mixing cured tobacco or cured and fermented tobacco with water or another solvent (e.g., ethanol) followed by removing the insoluble tobacco material. The tobacco extract may be further concentrated or purified. In some cases, select tobacco constituents can be removed. Nicotine can also be extracted from tobacco in the methods described in the following patents: U.S. Patent Nos. 2,162,738; 3,139,436; 3,396,735; 4,153,063; 4,448,208; and 5,487,792.

The nicotine can also be purchased from commercial sources, whether tobacco-derived or synthetic. In some cases, a lozenge provided herein can include a derivative of nicotine (e.g., a salt of nicotine).

Liquid nicotine can be pure, substantially pure, or diluted prior to mixing it with the soluble fiber. A diluting step is optional. In some cases, liquid nicotine is diluted to a concentration of between 1 weight percent and 75 weight percent prior to mixing the liquid nicotine with the soluble fiber. In some cases, liquid nicotine is diluted to a concentration of between 2 weight percent and 50 weight percent prior to mixing the liquid nicotine with the soluble fiber. In some cases, liquid nicotine is diluted to a concentration of between 5 weight percent and 25 weight percent prior to mixing the liquid nicotine with the soluble fiber. For example, liquid nicotine can be diluted to a concentration of about 10 weight percent prior to mixing the liquid nicotine with the soluble fiber.

### Amorphous, Non-Cross-Linked, Soluble Fibers

Amorphous, non-cross-linked, soluble fiber dissolves in water at ambient temperature. Insoluble fiber does not dissolve in water at ambient temperature. Amorphous, non-cross-linked, soluble fibers can attract water and form a gel. Not only are many amorphous, non-cross-linked soluble fibers safe for consumption, but some amorphous, non-cross-linked soluble fibers are used as a dietary supplement. As a dietary supplement, amorphous, non-cross-linked soluble fiber can slow down digestion and delay the emptying of a stomach. Instead of using amorphous, non-cross-linked soluble fiber as a mere additive, however, lozenges provided herein include a matrix of amorphous, non-cross-linked soluble fiber, which can dissolve to provide access to nicotine (and optionally one or more other ingredients included in the soluble-fiber matrix.)

Any suitable soluble fiber or combination of amorphous, non-cross-linked soluble fibers can be used to form a soluble-fiber matrix provided herein. Suitable amorphous, non-cross-linked soluble fibers include maltodextrin, psyllium, pectin, guar gum, gum arabic, inulin, arabinoxylans, cellulose, and many other plant components, such as resistant starch, resistant dextrins, lignin, pectins, beta-glucans, and oligosaccharides or a combination thereof. In some cases, a lozenge provided herein can include a amorphous, non-cross-linked, digestion resistant soluble fiber. An amorphous, non-cross-linked, digestion resistant soluble fiber can include starch linkages that remain undigested by enzymes of the human digestive tract. In some cases, a lozenge provided herein can include a digestion-resistant maltodextrin. In some cases, a digestion-resistant maltodextrin can be derived from maze. Suitable maltodextrins can include those that are soluble in water up to 70% at 20° C, have a viscosity of about 15 cps for a 30% solution at 30° C, a DE in the range of about 6-16, and contain random α-1,2, α-1,3, α-1,4, β -1,2, β -1,3 and β -1,4 glucose linkages in addition to the normal α-1,4 glucose linkages found in partially hydrolyzed starch. See, for example, US Patent Nos. 5,410,035; 5,380, 717. For example, Fibersol^{®}-2 is a maltodextrin of DE 6-10 processed from corn starch using hydrochloric acid and enzymes, which can be used as the soluble fiber in a lozenge provided herein. Fibersol^{®}-2 is partially indigestible because human digestive enzymes are incapable of digesting β 1,2, β 1,3 and β 1,6 glucose bonds. See, for example, US Patent No. 6,203,842. In addition to maize, other starch sources, such as potato, rice, wheat, barley, peas, beans, lentils, oats, or tapioca, can be processed to form amorphous, non-cross-linked, digestion resistant soluble fiber. An amorphous, non-cross-linked, digestion resistant soluble fiber includes starch linkages that cannot be hydrolyzed by enzymes of the human digestive tract. In some cases, suitable soluble fibers include Pinefibre C, Dexflow and Pineflow as discussed in U.S. Patent No. 5,236,719. The amorphous, non-cross-linked soluble fiber used in a lozenge provided herein can be GRAS by the Food and Drug Administration or another appropriate private, state, or national regulatory agency.

A lozenge body can include at least 40 weight percent of amorphous, non-cross-linked soluble fiber, at least 50 weight percent of amorphous, non-cross-linked soluble fiber, at least 60 weight percent of soluble, at least 70 weight percent of amorphous, non-cross-linked soluble fiber, at least 75 weight percent of amorphous, non-cross-linked soluble fiber, at least 80 weight percent of amorphous, non-cross-linked soluble fiber, at least 85 weight percent of amorphous, non-cross-linked soluble fiber, at least 90 weight percent of amorphous, non-cross-linked soluble fiber, or at least 95% weight percent of amorphous, non-cross-linked soluble fiber. In some cases, a lozenge body can include at least 40 weight percent maltodextrin, at least 50 weight percent maltodextrin, at least 60 weight percent maltodextrin, at least 70 weight percent maltodextrin, at least 75 weight percent maltodextrin, at least 80 weight percent maltodextrin, at least 85 weight percent maltodextrin, at least 90 weight percent maltodextrin, or at least 95 weight percent maltodextrin. In some cases, a lozenge body can include less than 95 weight percent maltodextrin, less than 90 weight percent maltodextrin, less than 85 weight percent maltodextrin, or less than 80 weight percent maltodextrin. In some cases, a lozenge body can include at least 40 weight percent digestion-resistant maltodextrin, at least 50 weight percent digestion-resistant maltodextrin, at least 60 weight percent digestion-resistant maltodextrin, at least 70 weight percent digestion-resistant maltodextrin, at least 75 weight percent digestion-resistant maltodextrin, at least 80 weight percent digestion-resistant maltodextrin, at least 85 weight percent digestion-resistant maltodextrin, at least 90 weight percent digestion-resistant maltodextrin, or at least 95 weight percent digestion-resistant maltodextrin.

### Tobacco

Tobacco plant tissue (e.g., tobacco particles) can be dispersed in a matrix of soluble fiber in a lozenge provided herein. As will be discussed below, tobacco plant tissue (e.g., tobacco particles) can be mixed with the molten mixture of soluble fiber during an extrusion process. Tobacco plant tissue can provide passages in the lozenge, which can permit certain tobacco constituents and/or additives within the lozenge to be released into an oral cavity when the lozenge is received in an oral cavity and exposed to saliva.

Suitable tobaccos include fermented and unfermented tobaccos. In addition to fermentation, the tobacco can be processed using other techniques. For example, tobacco can be processed by heat treatment (e.g., cooking, toasting), flavoring, enzyme treatment, expansion and/or curing. Both fermented and non-fermented tobaccos can be processed using these techniques. In other embodiments, the tobacco can be unprocessed tobacco. Specific examples of suitable processed tobaccos include dark air-cured, dark fire cured, burley, flue cured, and cigar filler or wrapper, as well as the products from the whole leaf stemming operation. In some embodiments, the tobacco fibers include up to 70% dark tobacco on a fresh weight basis. For example, tobacco can be conditioned by heating, sweating and/or pasteurizing steps as described in U.S. Publication Nos. 2004/0118422 or 2005/0178398. Fermenting typically is characterized by high initial moisture content, heat generation, and a 10 to 200 loss of dry weight. See, for example, U.S. Patent Nos. 4,528,993; 4,660,577; 4,848,373; and 5,372,149. In addition to modifying the aroma of the leaf, fermentation can change either or both the color and texture of a leaf. Also, during the fermentation process, evolution gases can be produced, oxygen can be taken up, the pH can change, and the amount of water retained can change. See, for example, U.S. Publication No. 2005/0178398 and Tso (1999, Chapter 1 in Tobacco, Production, Chemistry and Technology, Davis & Nielsen, eds., Blackwell Publishing, Oxford). Cured, or cured and fermented tobacco, can be further processed (e.g., cut, expanded, blended, milled or comminuted) prior to incorporation into the oral tobacco product. The tobacco, in some embodiments, is long cut fermented cured moist tobacco having an oven volatiles content of between 48 and 50 weight percent prior to mixing with the mouth-stable polymer and optionally flavorants and other additives.

The tobacco can, in some embodiments, be prepared from plants having less than 20 ug of DVT per cm² of green leaf tissue. For example, the tobacco fibers can be selected from the tobaccos described in U.S. Patent Publication No. 2008/0209586. Tobacco compositions containing tobacco from such low-DVT varieties exhibit improved flavor characteristics in sensory panel evaluations when compared to tobacco or tobacco compositions that do not have reduced levels of DVTs.

Green leaf tobacco can be cured using conventional means (e.g., flue-cured, barn-cured, fire-cured, air-cured or sun-cured). See, for example, Tso (1999, Chapter 1 in Tobacco, Production, Chemistry and Technology, Davis & Nielsen, eds., Blackwell Publishing, Oxford) for a description of different types of curing methods. Cured tobacco is usually aged in a wooden drum (i.e., a hogshead) or cardboard cartons in compressed conditions for several years (e.g., two to five years) at a moisture content ranging from 10% to about 25%. See U.S. Patent Nos. 4,516,590 and 5,372,149. Cured and aged tobacco then can be further processed. Further processing includes conditioning the tobacco under vacuum with or without the introduction of steam at various temperatures, pasteurization, and fermentation. Fermentation typically is characterized by high initial moisture content, heat generation, and a 10 to 200 loss of dry weight. See, for example, U.S. Patent Nos. 4,528,993, 4,660,577, 4,848,373, 5,372,149; U.S. Publication No. 2005/0178398; and Tso (1999, Chapter 1 in Tobacco, Production, Chemistry and Technology, Davis & Nielsen, eds., Blackwell Publishing, Oxford). Cure, aged, and fermented tobacco can be further processed (e.g., cut, shredded, expanded, or blended). See, for example, U.S. Patent Nos. 4,528,993; 4,660,577; and 4,987,907.

Tobacco plant tissue can be processed to a desired size (e.g., a desired particle size). In some cases, the tobacco fiber can be processed to have an average fiber size of less than 200 micrometers, less than 150 micrometers, less than 125 micrometers, less than 100 micrometers, less than 75 micrometers, less than 50 micrometers, less than 25 micrometers, less than 20 micrometers, or less than 10 micrometers. In some cases, tobacco plant tissue used in a lozenge provided herein can be processed to have an average particle size of at least 1 micrometer, at least 5 micrometers, at least 10 micrometers, at least 25 micrometers, at least 50 micrometers, at least 75 micrometers, at least 100 micrometers, at least 125 micrometers, or at least 150 micrometers. In some cases, tobacco plant tissue used in a lozenge provided herein can be processed to have an average particle size of between 25 and 125 micrometers. In some embodiments, the tobacco fibers include long cut tobacco, which can be cut or shredded into widths of about 10 cuts per inch up to about 110 cuts per inch and lengths of about 0.1 inches up to about 1 inch. Double cut tobacco fibers can have a range of particle sizes such that about 70% of the double cut tobacco fibers fall between the mesh sizes of 20 mesh and 80 mesh.

Tobacco plant tissue used in a lozenge provided herein can have a total oven volatiles content of about 1% by weight or greater; about 20% by weight or greater; about 40% by weight or greater; about 15% by weight to about 25% by weight; about 200 by weight to about 30% by weight; about 30% by weight to about 50% by weight; about 45% by weight to about 65% by weight; or about 50% by weight to about 60% by weight. Those of skill in the art will appreciate that "moist" tobacco typically refers to tobacco that has an oven volatiles content of between about 400 by weight and about 60% by weight (e.g., about 45% by weight to about 55% by weight, or about 50% by weight). As used herein, "oven volatiles" are determined by calculating the percentage of weight loss for a sample after drying the sample in a pre-warmed forced draft oven at 110 °C for 3.25 hours. The lozenge can have a different overall oven volatiles content than the oven volatiles content of the tobacco fibers used to make the oral tobacco product. The processing steps described herein can reduce or increase the oven volatiles content.

In some cases, the tobacco fibers include up to 70% dark tobacco on a fresh weight basis. For example, the tobacco can also be conditioned by heating, sweating and/or pasteurizing steps as described in U.S. Publication Nos. 2004/0118422 or 2005/0178398. Fermenting typically is characterized by high initial moisture content, heat generation, and a 10 to 200 loss of dry weight. See, for example, U.S. Patent Nos. 4,528,993; 4,660,577; 4,848,373; and 5,372,149. In addition to modifying the aroma of the leaf, fermentation can change either or both the color and texture of a leaf. Also, during the fermentation process, evolution gases can be produced, oxygen can be taken up, the pH can change, and the amount of water retained can change. See, for example, U.S. Publication No. 2005/0178398 and Tso (1999, Chapter 1 in Tobacco, Production, Chemistry and Technology, Davis & Nielsen, eds., Blackwell Publishing, Oxford). Cured or cured and fermented tobacco can be further processed (e.g., cut, expanded, blended, milled or comminuted) prior to incorporation into the oral tobacco product. The tobacco, in some embodiments, is long cut fermented cured moist tobacco having an oven volatiles content of between 48 and 50 weight percent prior to mixing with the mouth-stable polymer and optionally flavorants and other additives. See, for example, U.S. Patent Nos. 4,528,993; 4,660,577; 4,848,373; and 5,372,149. By processing the tobacco prior to extracting nicotine and other organoleptic components, the tobacco-derived nicotine may include ingredients that provide a favorable experience. The tobacco-derived nicotine can be obtained by mixing cured tobacco or cured and fermented tobacco with water or another solvent (e.g., ethanol), followed by removing the insoluble tobacco material. The tobacco extract may be further concentrated or purified. In some cases, select tobacco constituents can be removed. Nicotine can also be extracted from tobacco in the methods described in the following patents: U.S. Patent Nos. 2,162,738; 3,139,436; 3,396,735; 4,153,063; 4,448,208; and 5,487,792.

The tobacco can, in some embodiments, be prepared from plants having less than 20 ug of DVT per cm² of green leaf tissue. For example, the tobacco fibers can be selected from the tobaccos described in U.S. Patent Publication No. 2008/0209586. Tobacco compositions containing tobacco from such low-DVT varieties exhibit improved flavor characteristics in sensory panel evaluations when compared to tobacco or tobacco compositions that do not have reduced levels of DVTs.

Green leaf tobacco can be cured using conventional means (e.g., flue-cured, barn-cured, fire-cured, air-cured or sun-cured). See, for example, Tso (1999, Chapter 1 in Tobacco, Production, Chemistry and Technology, Davis & Nielsen, eds., Blackwell Publishing, Oxford) for a description of different types of curing methods. Cured tobacco is usually aged in a wooden drum (i.e., a hogshead) or cardboard cartons in compressed conditions for several years (e.g., two to five years) at a moisture content ranging from 10% to about 25%. See, U.S. Patent Nos. 4,516,590 and 5,372,149. Cured and aged tobacco then can be further processed. Further processing includes conditioning the tobacco under vacuum with or without the introduction of steam at various temperatures, pasteurization, and fermentation. Fermentation typically is characterized by high initial moisture content, heat generation, and a 10 to 200 loss of dry weight. See, for example, U.S. Patent Nos. 4,528,993, 4,660,577, 4,848,373, 5,372,149; U.S. Publication No. 2005/0178398; and Tso (1999, Chapter 1 in Tobacco, Production, Chemistry and Technology, Davis & Nielsen, eds., Blackwell Publishing, Oxford). Cure, aged, and fermented tobacco can be further processed (e.g., cut, shredded, expanded, or blended). See, for example, U.S. Patent Nos. 4,528,993; 4,660,577; and 4,987,907.

Tobacco plant tissue can be processed to a desired size (e.g., a desired particle size). In some cases, the tobacco fiber can be processed to have an average fiber size of less than 200 micrometers, less than 150 micrometers, less than 125 micrometers, less than 100 micrometers, less than 75 micrometers, less than 50 micrometers, less than 25 micrometers, less than 20 micrometers, or less than 10 micrometers. In some cases, tobacco plant tissue used in a lozenge provided herein can be processed to have an average particle size of at least 1 micrometer, at least 5 micrometers, at least 10 micrometers, at least 25 micrometers, at least 50 micrometers, at least 75 micrometers, at least 100 micrometers, at least 125 micrometers, or at least 150 micrometers. In some cases, tobacco plant tissue used in a lozenge provided herein can be processed to have an average particle size of between 25 and 125 micrometers. In some embodiments, the tobacco fibers include long cut tobacco, which can be cut or shredded into widths of about 10 cuts/inch up to about 110 cuts/inch and lengths of about 0.1 inches up to about 1 inch. Double cut tobacco fibers can have a range of particle sizes such that about 70% of the double cut tobacco fibers fall between the mesh sizes of 20 mesh and 80 mesh.

Tobacco plant tissue used in a lozenge provided herein can have a total oven volatiles content of about 1% by weight or greater; about 20% by weight or greater; about 40% by weight or greater; about 15% by weight to about 25% by weight; about 200 by weight to about 30% by weight; about 30% by weight to about 50% by weight; about 45% by weight to about 65% by weight; or about 50% by weight to about 60% by weight. Those of skill in the art will appreciate that "moist" tobacco typically refers to tobacco that has an oven volatiles content of between about 400 by weight and about 60% by weight (e.g., about 45% by weight to about 55% by weight, or about 50% by weight). As used herein, "oven volatiles" are determined by calculating the percentage of weight loss for a sample after drying the sample in a pre-warmed forced draft oven at 110 °C for 3.25 hours. The lozenge can have a different overall oven volatiles content than the oven volatiles content of the tobacco fibers used to make the oral tobacco product. The processing steps described herein can reduce or increase the oven volatiles content.

### Exhausted-tobacco fibers

A lozenge provided herein can include exhausted-tobacco fibers within a soluble-fiber matrix. As will be discussed below, the exhausted-tobacco fibers can be mixed with the soluble fiber prior to or during an extrusion process. Exhausted-tobacco fibers can provide passages in a soluble-fiber matrix, which can permit certain additives within a soluble-fiber matrix to be released into an oral cavity when a lozenge provided herein is received in an oral cavity and exposed to saliva. The additives can be absorbed in fiber-polymer matrix and/or form pockets within a soluble-fiber matrix, which can be accessed via the exhausted-tobacco fibers or as the soluble-fiber matrix dissolves. A lozenge provided herein can also include channels and pores formed in the exhausted-tobacco fibers. The water-soluble additives can be wicked by the exhausted-tobacco fibers.

Exhausted-tobacco fibers are derived from tobacco plant tissue. Exemplary species of tobacco include *N*. *rustica, N. tabacum, N. tomentosiformis,* and *N*. *sylvestris.* The exhausted-tobacco fibers can be obtained from any part of a tobacco plant, including the stems, leaves, or roots of a tobacco plant. The tobacco plant tissue is treated to remove at least 10 weight percent of the tobacco's soluble components, which can include alkaloids (e.g., nicotine) and nitrosamines. In some cases, the exhausted tobacco plant tissue can be treated to remove at least 25%, 40%, 50%, 60%, 70%, 75%, 80%, 85%, 90%, or 950, or 990 of the tobacco's soluble components. In some cases, the exhausted-tobacco fibers include less than 75%, less than 50%, less than 250, less than 10%, less than 50, or less than 1% of the nicotine normally found in tobacco plant tissue. In some cases, the exhausted-tobacco fibers include less than 75%, less than 50%, less than 250, less than 10%, less than 50, or less than 1% of the nitrosamines normally found in tobacco plant tissue. The treatment can also remove other soluble components of the tobacco plant tissue. In some cases, the exhausted tobacco can be obtained by washing tobacco plant tissue (e.g., tobacco stems) with slightly basic buffer solution. In some cases, exhausted tobacco can be obtained by treating the tobacco with supercritical fluids. For example, the exhausted tobacco can be obtained by the processes described in U.S. Patent No. 7,798,151.

Before or after treatment to remove at least some of the tobacco's soluble components, tobacco plant tissue can be treated by one or more conventional tobacco treating techniques, which may impact the flavor, aroma, color, and/or texture of the tobacco plant tissue. Some conventional tobacco treating techniques include fermentation, heat treating, enzyme treating, expanding, and curing. Exhausted-tobacco fibers can have the aroma of tobacco without contributing significantly to the components released by the exhausted tobacco oral product. Desired quantities of particular components can be added the exhausted tobacco oral product.

The exhausted-tobacco fibers can, in some cases, be prepared from plants having less than 20 ug of DVT per cm² of green leaf tissue. For example, the tobacco particles can be selected from the tobaccos described in U.S. Patent Publication No. 2008/0209586.

Exhausted-tobacco fibers can be processed to a desired size. In certain embodiments, the cellulosic fiber can be processed to have an average fiber size of less than 200 micrometers. In particular embodiments, the fibers are between 25 and 125 micrometers. In other embodiments, the fibers are processed to have a size of 75 micrometers or less. In still other embodiments, the exhausted-tobacco fibers can be cut or shredded into widths of about 10 cuts/inch up to about 110 cuts/inch and lengths of about 0.1 inches up to about 1 inch. Exhausted-tobacco fibers can also be cut twice to have a range of particle sizes such that about 70% of the exhausted-tobacco fibers fall between the mesh sizes of 20 mesh and 80 mesh. Additives can be absorbed in exhausted-tobacco fibers. In some cases, exhausted-tobacco fibers are hydrophilic, such that water-soluble additives can be wicked by the exhausted-tobacco fibers.

Exhausted-tobacco fibers can have a total oven volatiles content of about 0.50 or greater; about 10% by weight or greater; about 20% by weight or greater; about 40% by weight or greater; about 0.5% by weight to about 10% by weight; about 5% to 20% by weight; about 15% by weight to about 25% by weight; about 20% by weight to about 30% by weight; about 30% by weight to about 50% by weight; about 45% by weight to about 65% by weight; or about 50% by weight to about 60% by weight. As used herein, "oven volatiles" are determined by calculating the percentage of weight loss for a sample after drying the sample in a pre-warmed forced draft oven at 110 °C for 3.25 hours.

Exhausted-tobacco fibers can also be combined with non-tobacco cellulosic fibers. Suitable sources for non-tobacco cellulosic fibers include wood pulp, cotton, sugar beets, bran, citrus pulp fiber, switch grass and other grasses, Salix (willow), tea, and Populus (poplar). In some cases, the non-tobacco cellulosic fibers can be plant tissue comprising various natural flavors, sweeteners, or active ingredients.

### Additives

A variety of additives other than exhausted-tobacco fiber can be included in a lozenge provided herein. The additives can include alkaloids (e.g., nicotine), minerals, vitamins, dietary supplements, nutraceuticals, energizing agents, soothing agents, coloring agents, amino acids, chemesthic agent, antioxidants, food grade emulsifiers, pH modifiers, botanicals (e.g., green tea), teeth whitening (e.g., SHMP), therapeutic agents, sweeteners, and flavorants. In some cases, the additives can further include one or more non-nutritive sweeteners, one or more antioxidants, and one or more flavorants.

A soothing agent provides a soothing sensation to the throat and oral cavity. Suitable soothing agents include, without limitation, chamomile, lavender, jasmine, and the like. Suitable chemesthic ingredients provide, without limitation, hot, spicy, or cooling flavors such as mint, menthol, cinnamon, pepper, and the like.

Energizing ingredients or vitamins include, without limitation, caffeine, taurine, guarana, vitamin B6, vitamin B12, and the like. According to certain embodiments, a soluble fiber lozenge provided herein includes caffeine. A caffeinated oral product can include synthetic caffeine and/or coffee-bean-extracted caffeine. In some cases, a caffeinated oral product includes coffee flavors and sweeteners. In some cases, the fibers in a caffeinated oral product are coffee bean fibers. According to some embodiments, an oral product can include between 10 and 200 mg of caffeine.

The lozenges provided herein can also include vitamins, dietary minerals, other dietary supplements, and/or therapeutic agents. For example, suitable vitamins include Vitamins A, B1, B2, B6, C, D2, D3, E, F, and K. For example, an oral product 110 can include C-vitamins with or without the presence of caffeine. One or more dietary minerals could be included in an oral product with or without the use of other additives. Other dietary supplements and/or therapeutic agents can also be included as additives. Suitable dietary minerals include calcium (as carbonate, citrate, etc.) or magnesium (as oxide, etc.), chromium (usually as picolinate), and iron (as bis-glycinate).

In some cases, a lozenge provided herein includes a therapeutic agent that is preferably absorbed transbuccally (for example, so therapeutic agents do not pass into the blood stream if they are swallowed). Exemplary therapeutic agents that can be included in an oral product 110 provided herein can include Gerd, Buprenorphin, Nitroglycerin, Diclofenac, Fentanyl, Carbamazepine, Galantamine, Acyclovir, Polyamidoamine Nanoparticles, Chlorpheniramine, Testosterone, Estradiol, Progesterone, Calcitonin, Fluorouracil, Naltrexone, Odansetron, Decitabine, Selegiline, Lamotrigine, and Prochlorperazine. For example, an oral product 110 can include Buprenorphine and be used for pain treatment. In some cases, an oral product 110 can include Nitroglycerin and be used for Angina Pectoris treatment. Because of the release properties of a soluble fiber lozenge provided herein, therapeutic agents included therein can be released at a rate such that a majority of the therapeutic agent is absorbed transbuccally, rather than swallowed.

A lozenge provided herein can also include fillers such as starch, di-calcium phosphate, lactose, sorbitol, mannitol, and microcrystalline cellulose, calcium carbonate, dicalcium phosphate, calcium sulfate, clays, silica, sodium lauryl sulfate (SLS), glyceryl palmitostearate, sodium benzoate, sodium stearyl fumarate, talc, and stearates (e.g., Mg or K), waxes (e.g., glycerol monostearate, propylene glycol monostearate, and acetylated monoglycerides), stabilizers (e.g., ascorbic acid and monosterol citrate, BHT, or BHA), disintegrating agents (e.g., starch, sodium starch glycolate, cross caramellose, crosslinked PVP), pH stabilizers, or preservatives.

In some cases, with certain combinations of nicotine, sweeteners, and flavorants, lozenges provided herein may provide a flavor profile and tactile experience similar to certain tobacco products.

### Antioxidants

A lozenge 110 provided herein can include one or more antioxidants. Antioxidants can result in a significant reduction in the conversion of nicotine into nicotine-N-oxide when compared to nicotine products without antioxidants. In some cases, a lozenge provided herein can include 0.01 and 5.00 weight percent antioxidant, between 0.05 and 1.0 weight percent antioxidant, between 0.10 and 0.75 weigh percent antioxidant, or between 0.15 and 0.5 weight percent antioxidant. Suitable examples of antioxidants include ascorbyl palmitate (a vitamin C ester), BHT, ascorbic acid (Vitamin C), and sodium ascorbate (Vitamin C salt). In some cases, monosterol citrate, tocopherols, propyl gallate, tertiary butylhydroquinone (TBHQ), butylated hydroxyanisole (BHA), Vitamin E, or a derivative thereof can be used as the antioxidant. For example, ascorbyl palmitate can be the antioxidant in the formulations listed in Table I. Antioxidants can be incorporated into the soluble-fiber matrix (e.g., maltodextrin) during a mixing process (e.g., added to an extruder mixing the ingredients).

In some cases, the amount of filler in a soluble fiber lozenge provided herein is limited to less than 10 weight percent in sum. In some cases, the amount of filler in a soluble fiber lozenge provided herein is limited to be less than 5 weight percent in sum. In some cases, the fillers are mouth stable. In some cases, the fillers can dissolve or disintegrate during use and thus result in an oral product that becomes more pliable during use.

In some cases, humectants can be added to help maintain the moisture levels in a soluble fiber lozenge provided herein. Examples of humectants include glycerin and propylene glycol. In some cases, anti-microbial agents can be added to prevent spoilage and to lengthen shelf-life.

### Sweeteners

A variety of synthetic and/or natural sweeteners can be used as additives in a lozenge 110 provided herein. Suitable natural sweeteners include sugars (for example, monosaccharides, disaccharides, and/or polysaccharide sugars, and/or mixtures of two or more sugars). In some cases, a lozenge 110 provided herein includes one or more of the following: sucrose or table sugar; honey or a mixture of low molecular weight sugars not including sucrose; glucose or grape sugar or corn sugar or dextrose; molasses; corn sweetener; corn syrup or glucose syrup; fructose or fruit sugar; lactose or milk sugar; maltose or malt sugar or maltobiose; sorghum syrup; mannitol or manna sugar; sorbitol or d-sorbite or d-sobitol; fruit juice concentrate; and/or mixtures or blends of one or more of these ingredients. A lozenge provided herein an also include non-nutritive sweeteners. Suitable non-nutritive sweeteners include: stevia, saccharin, aspartame, sucralose, or acesulfame potassium.

### Flavorants

The lozenge provided herein can optionally include one or more flavorants as an additive. The flavorants can be natural or artificial. For example, suitable flavorants include wintergreen, cherry and berry type flavorants, various liqueurs and liquors (such as Dramboui, bourbon, scotch, and whiskey), spearmint, peppermint, lavender, cinnamon, cardamom, apium graveolents, clove, cascarilla, nutmeg, sandalwood, bergamot, geranium, honey essence, rose oil, vanilla, lemon oil, orange oil, Japanese mint, cassia, caraway, cognac, jasmin, chamomile, menthol, ylang ylang, sage, fennel, pimenta, ginger, chai, anise, coriander, coffee, liquorish, and mint oils from a species of the genus Mentha, and encapsulated flavors. Mint oils useful in particular embodiments of a lozenge provided herein include spearmint and peppermint. Synthetic flavorants can also be used. In some cases, a combination of flavorants can be combined to imitate a tobacco flavor. The particular combination of flavorants can be selected from flavorants that are GRAS in a particular country, such as the United States. Flavorants can also be included in the lozenge as encapsulated flavorants.

In some cases, the flavorants in a lozenge provided herein are limited to less than 20 weight percent in sum. In some cases, the flavorants in lozenge 110 are limited to be less than 10 weight percent in sum. For example, certain flavorants can be included in the lozenge 110 in amounts of about 1 weight percent to 5 weight percent.

### Cellulosic Fibers

The lozenges provided herein can optionally include cellulosic fibers within the soluble-fiber matrix. In some cases, lozenges 110 provided herein are free of cellulosic fibers. As used here, "free of cellulosic fibers" means that a lozenge provided herein has less than 0.5 weight percent cellulosic fibers. In some cases, lozenges 110 provided herein include up to 40 weight percent cellulosic fibers. In some cases, lozenges 110 provided herein include up to 10 weight percent cellulosic fibers. Cellulosic fibers can be mixed with molten mixtures including soluble fibers to form a lozenge provided herein. Additives can be absorbed in cellulosic fibers. In some cases, the fibers are hydrophilic such that water-soluble additives can be wicked by the fibers. Cellulosic fibers can be derived from plant tissue. Suitable sources for cellulosic fibers include wood pulp, cotton, sugar beets, bran, citrus pulp fiber, switch grass and other grasses, Salix (willow), tea, and Populus (poplar). In some cases, the cellulosic fibers can be plant tissue comprising various natural flavors, sweeteners, or active ingredients. Cellulosic fibers can have a variety of dimensions. In some cases, cellulosic fibers in a lozenge can have an average fiber length of less than 200 micrometers. In some cases, cellulosic fibers in a lozenge 110 provided herein have an average fiber length of between 25 and 125 micrometers. In some cases, cellulosic fibers are processed to have an average length of 75 micrometers or less.

### Plasticizers

A lozenge 110 provided herein also includes one or more plasticizers. Plasticizers can soften the final lozenge and thus increase its flexibility. Suitable plasticizers include propylene glycol, triacetin, glycerin, vegetable oil, partially hydrogenated oil, and medium chain triglycerides. Esters of polycarboxylic acids with linear or branched aliphatic alcohols of moderate chain length can also be used as plasticizers. Moreover, plasticizers can facilitate the extrusion processes described below. The lozenge 110 includes between 0.05 and 10 weight percent plasticizer. A lozenge 110 provided herein can include between 1 and 8 weight percent plasticizer, or between 2 and 4 weight percent plasticizer.

### Other Additives

A lozenge provided herein also includes an additive. For example, the additive can include non-nicotine alkaloids, dietary minerals, vitamins, dietary supplements, therapeutic agents, and fillers. For example, a lozenge 110 provided herein can include C-vitamins. One or more dietary minerals could be included in a lozenge with or without the use of other additives. Other dietary supplements and/or therapeutic agents can also be included as additives.

A lozenge provided herein can also include fillers such as starch, di-calcium phosphate, lactose, sorbitol, mannitol, and microcrystalline cellulose, calcium carbonate, dicalcium phosphate, calcium sulfate, clays, silica, sodium lauryl sulfate (SLS), glyceryl palmitostearate, sodium benzoate, sodium stearyl fumarate, talc, stearates (e.g., in some cases, the amount of filler in the lozenge 110 is limited to less than 10 weight percent in sum.) In some cases, the amount of filler in the lozenge 110 is limited to be less than 5 weight percent in sum. In some cases, the fillers are mouth stable. In some cases, the fillers can dissolve or disintegrate during use and thus result in a lozenge that becomes more pliable during use.

### Production and Example

The lozenge 110 can be produced by forming a molten mixture of amorphous, non-cross-linked soluble fiber, water, nicotine, and optionally other ingredients, such as exhausted-tobacco fibers and/or one or more additives under controlled heating conditions such that a solution or dispersion of amorphous, non-cross-linked soluble fiber is formed without degrading the nicotine or the soluble fiber. In some cases, a temperature of the molten mixture is maintained at a temperature below 150 °C for a period of time (e.g., a residence time between 5 to 10 minutes or less during the mixing.) In some cases, a temperature of the molten mixture is maintained at a temperature below 200 °C for a period of time (e.g., a residence time between 5 to 10 minutes or less during the mixing.) The molten mixture is then portioned into individual lozenges. Unlike many traditional lozenges, sugar and sugar alcohols are not required to obtain a firm smooth-dissolving texture in processes provided herein. Traditional lozenges can rely on the crosslinking of sugars or sugar alcohols due to caramelization caused by heating to caramelization temperatures. Caramelization temperatures, however, can degrade nicotine and certain additives or negatively impact one or more sensorial characteristics. A matrix of amorphous, non-cross-linked, soluble fiber, however, can provide a suitable dissolution time when placed in an adult consumer's mouth

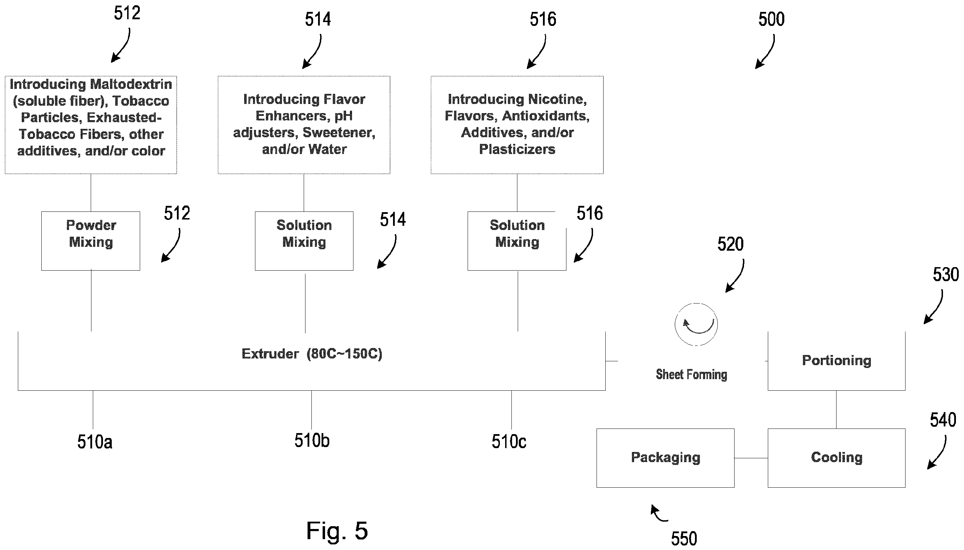

A molten mixture can be mixed and heated in any suitable but controlled method. In some cases, such as shown in Figure 5, ingredients for a molten mixture can be combined in an extruder and mixed in a continuous extrusion process. Unlike a traditional cooking method for many typical lozenges, a lozenge provided herein can have attributes precisely controlled by extruder operation parameters, such as feed rate, barrel temperature profile, screw design, rpms, etc.

Referring to Figure 5, an exemplary method for making lozenges provided herein can include adding dry ingredients 512 of amorphous, non-cross-linked soluble fiber (e.g., maltodextrin or exhausted-tobacco fibers, tobacco particles, and/or color) to a first station 510a of an extruder 510; adding a first group of solution ingredients including water, and sweetener, pH adjusters, and flavor enhancers at a second station 510b; and adding a second group of ingredients 516, including nicotine and/or one or more additives such as flavors, antioxidants and/or plasticizers at a third station 510c. A mixing extruder 510 can include multiple controlled stages to be maintained at a predetermined temperature over a residence time of five to ten minutes or less during the mixing. As shown in Figure 5, extruder 510 can include stages having temperatures ranging between 80 °C and 150 °C. In some cases, the extruder 510 can include stages having temperatures ranging between 80 °C and 200 °C. For example, dry ingredients 512 and a first group of solution ingredients 514 can be mixed in a first stage of extruder 510 at a temperature of between 100 °C and 120°C while one or more subsequent stages can have a higher temperature (e.g., between 120 °C and 150 °C). For example, dry ingredients 512 and a first group of solution ingredients 514 can be mixed in a first stage of extruder 510 at a temperature of between 100 °C and 115°C while one or more subsequent stages can have a higher temperature (e.g., between 120 °C and 200 °C). Second group of ingredients, including nicotine and one or more ingredients such as tobacco and/or one or more additives, can be added downstream of the mixture of water with the soluble fiber. Adding certain ingredients downstream can limit degradation of certain ingredients (e.g., nicotine or flavors) due to exposure to heat. In some cases, the extrudate can have a total residence time of between 5 and 10 minutes in extruder 510.

A glass transition temperature (Tg) of molten mixture used to make a lozenge provided herein can range from 50 °C to 120 °C (i.e., about 122 °F to about 248 °F). Figure 2, discussed above, is a DSC Chart for an exemplary molten mixture that has a Tg range of between 50 °C and 120 °C. Figure 2A depicts the modulated DSC for an exemplary mixture, provided herein, which shows a lozenge body that has a Tg of 98 °C.

Water content of a lozenge provided herein can be controlled in the extrusion process to ensure that the molten mixture has a glass transition temperature of greater than human body temperature. In some cases, a molten mixture can have a water content of less than 15 weight percent. In some cases, water content in a lozenge provided herein ranges from 2 weight percent to 15 weight percent. In some cases, water content in a lozenge provided herein ranges from 2 weight percent to 10 weight percent. In some cases, water content in a lozenge provided herein ranges from 2 weight percent to 20 weight percent.

After passing through the extruder, a molten mixture provided herein can have a temperature between its glass transition temperature and 150 °C. In some cases, a molten mixture provided herein can have a temperature between its glass transition temperature and 200 °C or a molten mixture provided herein can have a temperature of between its glass transition temperature and 150 °C. In some cases, a molten mixture provided herein can have a temperature of between its glass transition temperature and 150 °C.

In some cases, a molten mixture of between 85 and 95 weight percent or between 85 and 90 weight percent digestion-resistant maltodextrin can reach a maximum temperature in an extruder of between 100 °C and 150 °C and exit the extruder at that temperature or lower. In some cases, a molten mixture of between 85 and 90 weight percent digestion-resistant maltodextrin can reach a maximum temperature in an extruder of between 80 °C and 110 °C, between 100 °C and 150 °C, or between 100 °C and 200 °C, and exit the extruder at that temperature or lower. Because a molten mixture can remain above its glass transition temperature as it exits the extruder, the molten mixture can be reshaped after it exits the extruder. Molten mixture can pass onto a conveyor and move through a sheet forming apparatus 520. Sheet forming apparatus 520 can press the molten mixture into a sheet having a predetermined thickness. For example, a predetermined thickness can be between 2 mm and 10 mm or between 1 mm and 25 mm, can be between 2 mm and 10 mm, or can be between 1 mm and 10 mm

Individual lozenges 110 can be cut from a sheet of molten mixture in portioning station 530. In some cases, a stamping die can cut one or more individual lozenges 110 to form a sheet. In some cases, a stamping die can press one or both sides of a sheet to both cut a lozenge and reshape edges to form rounded edges on the lozenges, such as those shown in Figure 1. Cutting individual lozenges 110 can occur when the molten mixture is still above its Tg. Individual lozenges 110 can be cooled in a cooling station 540 and packaged in a packaging station 550.

In addition to extrusion, there are other methods for mixing and carefully controlling the temperature of a molten mixture used to form lozenges provided herein.

### Other Embodiments

It is to be understood that, while the invention has been described herein in conjunction with a number of different aspects, the foregoing description of the various aspects is intended to illustrate and not limit the scope of the invention, which is defined by the scope of the appended claims. Other aspects, advantages, and modifications are within the scope of the following claims.

Disclosed are methods and compositions that can be used for, can be used in conjunction with, can be used in preparation for, or are products of the disclosed methods and compositions. These and other materials are disclosed herein, and it is understood that combinations, subsets, interactions, groups, etc. of these methods and compositions are disclosed. That is, while specific reference to each various individual and collective combinations and permutations of these compositions and methods may not be explicitly disclosed, each is specifically contemplated and described herein. For example, if a particular composition of matter or a particular method is disclosed and discussed and a number of compositions or methods are discussed, each and every combination and permutation of the compositions and the methods are specifically contemplated unless specifically indicated to the contrary. Likewise, any subset or combination of these is also specifically contemplated and disclosed.

## Claims

1. A lozenge comprising:
a non-porous body that is wholly receivable in an oral cavity, the body having a glass transition temperature ranging from 50°C to 120°C the body including,
a matrix including,
amorphous, non-cross-linked soluble fibers present in the body in an amount greater than or equal to 40 weight percent of the body,
a plasticizer in an amount ranging from 0.05 weight percent to 10 weight percent, the plasticizer being dispersed in the matrix, and the plasticizer including propylene glycol, triacetin, glycerin, vegetable oil, partially hydrogenated oil, triglycerides, or any combination thereof,
water in an amount ranging from 2 weight percent to 15 weight percent, and
an additive dispersed in the matrix,
wherein the body is free of cellulosic fibers.

2. The lozenge of claim 1, wherein the soluble fibers comprise a maltodextrin, psyllium, inulin, an arabinoxylan, resistant starch, a resistant dextrin, lignin, a pectin, a beta-glucan, an oligosaccharide, or any combination thereof.

3. The lozenge of claim 1, further comprising:
a coating on at least a portion of a surface of the body.

4. The lozenge of claim 3, wherein the coating comprises a dissolvable film.

5. The lozenge of claim 3 or claim 4, wherein the coating comprises a mouth-stable material.

6. The lozenge of one or the claims 3 to 5, wherein the coating comprises at least one of carnauba wax, beeswax, gelatin, acetylated monoglyceride, starch, zein, shellac, ethyl cellulose, methylcellulose, hydroxypropyl methylcellulose, carboxymethyl cellulose or any combination thereof.

7. The lozenge of claim 1, wherein the additive comprises minerals, vitamins, dietary supplements, nutraceuticals, energizing agents, soothing agents, amino acids, chemesthetic agents, antioxidants, botanicals, teeth whitening agents, therapeutic agents, or any combination thereof.

8. The lozenge of claim 1, wherein
the body is configured to dissolve when the body is exposed to saliva, and
the additive is configured to be released from the body as the body dissolves.

9. The lozenge of claim 1, wherein the body includes less than 25 weight percent of sugars and sugar alcohols.

10. The lozenge of claim 1, wherein the body is free of sugars and sugar alcohols.

11. The lozenge of claim 1, wherein the matrix is formed from a molten mixture heated to a temperature of between 80°C and 110°C.

12. A lozenge comprising:
a non-porous body that is wholly receivable in an oral cavity, the body including,
a matrix including,
an amount greater than or equal to 40 weight percent of amorphous, non-cross-linked, digestion-resistant soluble fibers,
water in an amount ranging from 2 weight percent to 15 weight percent of the body, and
a plasticizer in an amount ranging from 0.5 weight percent to 10 weight percent, the plasticizer being dispersed in the matrix, and the plasticizer including propylene glycol, triacetin, glycerin, vegetable oil, partially hydrogenated oil, triglycerides, triacetin, or any combination thereof; and
an additive dispersed in the matrix.

13. The lozenge of claim 12, wherein the body has a glass transition temperature ranging from 50°C to 120°C.

14. The lozenge of claim 12, further comprising:
a coating on at least a portion of a surface of the body, the coating including a dissolvable film.

15. The lozenge of claim 12, wherein
the body is configured to dissolve when the body is exposed to saliva, and the additive is configured to be released from the body as the body dissolves.

16. The lozenge of claim 12, wherein the soluble fibers comprise a maltodextrin, psyllium, inulin, an arabinoxylan, cellulose, resistant starch, a resistant dextrin, lignin, a pectin, a beta-glucan, an oligosaccharide, or any combination thereof.

17. The lozenge of claim 12, wherein the matrix is formed from a molten mixture heated to a temperature of between 80°C and 110°C.

18. The lozenge of claim 12, wherein the digestion resistant soluble fibers comprise maltodextrin.

## Patentansprüche

1. Pastille, umfassend:
einen nicht-porösen Körper, der im Ganzen in einer Mundhöhle aufnehmbar ist, wobei der Körper eine Glasübergangstemperatur im Bereich von 50 °C bis 120 °C aufweist, wobei der Körper einschließt:
eine Matrix, die einschließt:
amorphe, nicht-vernetzte lösliche Fasern, die in einer Menge von mindestens 40 Gewichtsprozent des Körpers in dem Körper vorhanden sind,
einen Weichmacher in einer Menge in einem Bereich von 0,05 Gewichtsprozent bis 10 Gewichtsprozent, wobei der Weichmacher in der Matrix dispergiert ist und der Weichmacher Propylenglykol, Triacetin, Glycerin, Pflanzenöl, teilgehärtetes Öl, Triglyceride oder irgendeine Kombination davon einschließt,
Wasser in einer Menge in einem Bereich von 2 Gewichtsprozent bis 15 Gewichtsprozent und
einen Zusatz, der in der Matrix dispergiert ist,
wobei der Körper frei ist von cellulosischen Fasern.

2. Pastille nach Anspruch 1, wobei die löslichen Fasern ein Maltodextrin, Psyllium, Inulin, ein Arabinoxylan, resistente Stärke, ein resistentes Dextrin, Lignin, ein Pektin, ein Beta-Glucan, ein Oligosaccharid oder irgendeine Kombination davon umfassen.

3. Pastille nach Anspruch 1, ferner umfassend:
eine Beschichtung auf zumindest einem Abschnitt einer Oberfläche des Körpers.

4. Pastille nach Anspruch 3, wobei die Beschichtung einen auflösbaren Film umfasst.

5. Pastille nach Anspruch 3 oder 4, wobei die Beschichtung ein mundstabiles Material umfasst.

6. Pastille nach einem der Ansprüche 3 bis 5, wobei die Beschichtung mindestens eines umfasst von Carnaubawachs, Bienenwachs, Gelatine, acetyliertem Monoglycerid, Stärke, Zein, Schellack, Ethylcellulose, Methylcellulose, Hydroxypropylmethylcellulose, Carboxymethylcellulose oder irgendeine Kombination davon.

7. Pastille nach Anspruch 1, wobei der Zusatz Mineralien, Vitamine, Nahrungsergänzungsmittel, Nutrazeutika, anregende Wirkstoffe, lindernde Wirkstoffe, Aminosäuren, chemesthetische Wirkstoffe, Antioxidanzien, botanische Substanzen, Zahnaufheller, Therapeutika oder irgendeine Kombination davon umfasst.

8. Pastille nach Anspruch 1, wobei
der Körper ausgebildet ist, um sich aufzulösen, wenn der Körper Speichel ausgesetzt wird, und
der Zusatz ausgebildet ist, um aus dem Körper freigesetzt zu werden, wenn sich dieser auflöst.

9. Pastille nach Anspruch 1, wobei der Körper weniger als 25 Gewichtsprozent Zucker und Zuckeralkohole einschließt.

10. Pastille nach Anspruch 1, wobei der Körper frei ist von Zucker und Zuckeralkoholen.

11. Pastille nach Anspruch 1, wobei die Matrix aus einer auf eine Temperatur zwischen 80 °C und 110 °C erwärmten geschmolzenen Mischung gebildet ist.

12. Pastille, umfassend:
einen nicht-porösen Körper, der im Ganzen in einer Mundhöhle aufnehmbar ist wobei der Körper einschließt:
eine Matrix, die einschließt:
eine Menge von größer oder gleich 40 Gewichtsprozent an amorphen, unvernetzten, gegen Verdauung beständigen löslichen Fasern,
Wasser in einer Menge in einem Bereich von 2 Gewichtsprozent bis 15 Gewichtsprozent des Körpers und
einen Weichmacher in einer Menge in einem Bereich von 0,5 Gewichtsprozent bis 10 Gewichtsprozent, wobei der Weichmacher in der Matrix dispergiert ist, und der Weichmacher Propylenglykol, Triacetin, Glycerin, Pflanzenöl, teilgehärtetes Öl, Triglyceride, Triacetin oder irgendeine Kombination davon einschließt, und
einen in der Matrix dispergierten Zusatz.

13. Pastille nach Anspruch 12, wobei der Körper eine Glasübergangstemperatur in einem Bereich von 50 °C bis 120 °C aufweist.

14. Pastille nach Anspruch 12, ferner umfassend:
eine Beschichtung auf zumindest einem Abschnitt einer Oberfläche des Körpers, wobei die Beschichtung einen auflösbaren Film einschließt.

15. Pastille nach Anspruch 12, wobei
der Körper ausgebildet ist, um sich aufzulösen, wenn der Körper Speichel ausgesetzt wird, und der Zusatz ausgebildet ist, um aus dem Körper freigesetzt zu werden, wenn sich der Körper auflöst.

16. Pastille nach Anspruch 12, wobei die löslichen Fasern ein Maltodextrin, Psyllium, Inulin, ein Arabinoxylan, Cellulose, resistente Stärke, ein resistentes Dextrin, Lignin, ein Pektin, ein Beta-Glucan, ein Oligosaccharid oder irgendeine Kombination davon umfassen.

17. Pastille nach Anspruch 12, wobei die Matrix aus einer auf eine Temperatur zwischen 80 °C und 110 °C erwärmten geschmolzenen Mischung gebildet ist.

18. Pastille nach Anspruch 12, wobei die gegen Verdauung beständigen löslichen Fasern Maltodextrin umfassen.

## Revendications

1. Pastille comprenant :
un corps non poreux qui peut être reçu entièrement dans une cavité orale, le corps ayant une température de transition vitreuse comprise entre 50 °C et 120 °C, le corps comprenant,
une matrice comprenant,
des fibres solubles non réticulées amorphes présentes dans le corps dans une proportion supérieure ou égale à 40 pour cent en poids du corps,
un plastifiant dans une proportion comprise entre 0,05 pour cent en poids et 10 pour cent en poids, le plastifiant étant dispersé dans la matrice, et le plastifiant comprenant du propylène glycol, de la triacétine, de la glycérine, de l'huile végétale, de l'huile partiellement hydrogénée, des triglycérides ou n'importe quelle combinaison de ceux-ci,
de l'eau dans une proportion comprise entre 2 pour cent en poids et 15 pour cent en poids, et
un additif dispersé dans la matrice,
dans laquelle le corps est exempt de fibres cellulosiques.

2. Pastille selon la revendication 1, dans laquelle les fibres solubles comprennent une maltodextrine, du psyllium, de l'inuline, un arabinoxylane, de l'amidon résistant, une dextrine résistante, de la lignine, une pectine, un bêta-glucane, un oligosaccharide ou n'importe quelle combinaison de ceux-ci.

3. Pastille selon la revendication 1, comprenant en outre :
un enrobage sur au moins une partie d'une surface du corps.

4. Pastille selon la revendication 3, dans laquelle l'enrobage comprend une pellicule soluble.

5. Pastille selon la revendication 3 ou la revendication 4, dans laquelle l'enrobage comprend un matériau stable dans la bouche.

6. Pastille selon l'une des revendications 3 à 5, dans laquelle l'enrobage comprend au moins un parmi la cire de carnauba, la cire d'abeille, la gélatine, le monoglycéride acétylé, l'amidon, la zéine, la gomme-laque, l'éthylcellulose, la méthylcellulose, l'hydroxypropylméthylcellulose, la carboxyméthylcellulose ou n'importe quelle combinaison de ceux-ci.

7. Pastille selon la revendication 1, dans laquelle l'additif comprend des minéraux, des vitamines, des suppléments diététiques, des nutraceutiques, des agents énergisants, des agents calmants, des acides aminés, des agents chimesthétiques, des antioxydants, des agents botaniques, des agents de blanchiment des dents, des agents thérapeutiques ou n'importe quelle combinaison de ceux-ci.

8. Pastille selon la revendication 1, dans laquelle
le corps est conçu pour se dissoudre lorsque le corps est exposé à de la salive, et
l'additif est conçu pour être libéré du corps à mesure que le corps se dissout.

9. Pastille selon la revendication 1, dans laquelle le corps comprend moins de 25 pour cent en poids de sucres et d'alcools de sucres.

10. Pastille selon la revendication 1, dans laquelle le corps est exempt de sucres et d'alcools de sucres.

11. Pastille selon la revendication 1, dans laquelle la matrice est formée à partir d'un mélange fondu chauffé à une température comprise entre 80 °C et 110 °C.

12. Pastille comprenant :
un corps non poreux qui peut être reçu entièrement dans une cavité orale, le corps comprenant,
une matrice comprenant,
une proportion supérieure ou égale à 40 pour cent en poids de fibres solubles non réticulées amorphes résistant à la digestion,
de l'eau dans une proportion comprise entre 2 pour cent en poids et 15 pour cent en poids du corps, et
un plastifiant dans une proportion comprise entre 0,5 pour cent en poids et 10 pour cent en poids, le plastifiant étant dispersé dans la matrice, et le plastifiant comprenant du propylène glycol, de la triacétine, de la glycérine, de l'huile végétale, de l'huile partiellement hydrogénée, des triglycérides, de la triacétine ou n'importe quelle combinaison de ceux-ci ; et
un additif dispersé dans la matrice.

13. Pastille selon la revendication 12, dans laquelle le corps a une température de transition vitreuse comprise entre 50 °C et 120 °C.

14. Pastille selon la revendication 12, comprenant en outre :
un enrobage sur au moins une partie d'une surface du corps, l'enrobage comprenant une pellicule soluble.

15. Pastille selon la revendication 12, dans laquelle
le corps est conçu pour se dissoudre lorsque le corps est exposé à de la salive, et l'additif est conçu pour être libéré du corps à mesure que le corps se dissout.

16. Pastille selon la revendication 12, dans laquelle les fibres solubles comprennent une maltodextrine, du psyllium, de l'inuline, un arabinoxylane, de la cellulose, de l'amidon résistant, une dextrine résistante, de la lignine, une pectine, un bêta-glucane, un oligosaccharide ou n'importe quelle combinaison de ceux-ci.

17. Pastille selon la revendication 12, dans laquelle la matrice est formée à partir d'un mélange fondu chauffé à une température comprise entre 80 °C et 110 °C.

18. Pastille selon la revendication 12, dans laquelle les fibres solubles résistant à la digestion comprennent de la maltodextrine.
